# EUROPEAN PATENT APPLICATION

(11) **EP 4 772 635 A1**
(43) Date of publication of application: **08.07.2026**
(21) Application number: 24858726.3
(22) Date of filing: 30.08.2024
(51) Int. Cl.: C12N 15/113, A61P 25/28

(54) **RNAI AGENT TARGETING APP AND PHARMACEUTICAL USE THEREOF**

(30) Priority: 31.08.2023 CN 202311111515; 23.01.2024 CN 202410092623
(71) Applicant: Tuojie Biotech (Shanghai) Co., Ltd., Shanghai 201203 (CN)
(72) Inventor: LIU, Min, Shanghai 201203 (CN); CHEN, Di, Shanghai 201203 (CN); LIN, Xiaoyan, Shanghai 201203 (CN); LI, Yunfei, Shanghai 201203 (CN); WANG, Yanhui, Shanghai 201203 (CN)
(74) Representative: Dragotti & Associati S.P.A.
(86) International application number: PCT/CN2024/115823
(87) International publication number: WO 2025/045195

(57) **Abstract**

The present invention relates to an RNAi agent targeting APP and the pharmace utical use thereof. Specifically, the present invention relates to an RNAi agent tar geting APP, a pharmaceutical composition, and the pharmaceutical use thereof. T he present invention further relates to a pharmaceutical composition, cell or kit c ontaining the RNAi agent, and a method involving using the RNAi agent for tre ating and/or preventing a related disorder in a subject.

## Description

### TECHNICAL FIELD

The present disclosure pertains to the field of biopharmaceuticals and particularly relates to an RNAi agent targeting amyloid precursor protein (APP), a pharmaceutical composition comprising same, and pharmaceutical use thereof.

### BACKGROUND

The amyloid precursor protein (APP) gene encodes a membrane protein that is expressed in neurons and glia. APP is cleaved by α-secretase to produce sAPPα, which is soluble and non-neurotoxic. APP is cleaved by β-secretase and γ-secretase to produce Aβ polypeptides, which are hydrophobic and prone to aggregation and are secreted extracellularly. Aβ40 and Aβ42 are two common subtypes and, as major components of amyloid plaques, are believed to be associated with the development and progression of Alzheimer's disease (AD). In addition, both Alzheimer's disease and cerebral amyloid angiopathy in patients with Down syndrome are characterized by abnormal accumulation of amyloid plaques.

Using a drug that can selectively and effectively inhibit APP to inhibit the expression and/or activity of APP, thereby blocking or inhibiting the production and/or levels of Aβ, would help prevent or treat various APP-associated diseases. Such drugs, methods, and pharmaceutical compositions can be used to ameliorate at least one symptom or hallmark of neurodegenerative diseases, including cognitive disorders, such as declines in memory and speech; behavioral and psychological symptoms, such as apathy and lack of motivation, gait disturbances, and epileptic seizures; and progressive dementia and abnormal amyloid deposition. Neurodegenerative diseases include sporadic Alzheimer's disease, hereditary/familial Alzheimer's disease, Down syndrome, frontotemporal dementia, and cerebral amyloid angiopathy.

Currently, there is a lack of acceptable methods for treating such neurodegenerative diseases. Therefore, there is a need for drugs that selectively and effectively inhibit or regulate APP gene expression, in order to effectively treat subjects with APP-associated diseases. The objective of the present disclosure is to provide a method for treating such diseases.

### SUMMARY

The present disclosure provides an RNAi agent targeting APP.

In some embodiments, the present disclosure provides an RNAi agent comprising a sense strand and an antisense strand that form a double-stranded region, wherein:
the sense strand comprises a sequence differing from any one of the nucleotide sequences of SEQ ID NO: 1 to SEQ ID NO: 11 and SEQ ID NO: 23 to SEQ ID NO: 65 by no more than 3 (e.g., 0, 1, 2, or 3) nucleotides, and comprises at least 15 (e.g., 16, 17, 18, 19, 20, or 21) contiguous nucleotides;
the antisense strand comprises a sequence differing from any one of the nucleotide sequences of SEQ ID NO: 11 or SEQ ID NO: 22 and SEQ ID NO: 66 to SEQ ID NO: 108 by no more than 3 (e.g., 0, 1, 2, or 3) nucleotides, and comprises at least 15 (e.g., 16, 17, 18, 19, 20, or 21) contiguous nucleotides.

In some embodiments, the antisense strand is at least partially reverse complementary to a target sequence to mediate RNA interference. In some embodiments, there are no more than 5, no more than 4, no more than 3, no more than 2, or no more than 1 mismatch between the antisense strand and the target sequence. In some embodiments, the antisense strand is fully reverse complementary to the target sequence.

In some embodiments, the sense strand is at least partially reverse complementary to the antisense strand to form a double-stranded region. In some embodiments, there are no more than 5, no more than 4, no more than 3, no more than 2, or no more than 1 mismatch between the sense strand and the antisense strand. In some embodiments, the sense strand is fully reverse complementary to the antisense strand.

In some embodiments, the RNAi agent of the present disclosure comprises one or two blunt ends.

In some specific embodiments, each strand of the RNAi agent independently comprises an overhang comprising 1 to 2 unpaired nucleotides.

In some embodiments, the RNAi agent of the present disclosure comprises an overhang at the 3' end of the antisense strand of the RNAi agent.

In some embodiments, the sense strand and the antisense strand each independently have 16 to 35, 16 to 34, 17 to 34, 17 to 33, 18 to 33, 18 to 32, 18 to 31, 18 to 30, 18 to 29, 18 to 28, 18 to 27, 18 to 26, 18 to 25, 18 to 24, 18 to 23, 19 to 25, 19 to 24, or 19 to 23 nucleotides (e.g., 19, 20, 21, 22, or 23 nucleotides).

In some embodiments, the sense strand and the antisense strand are identical or different in length; the sense strand is 19-23 nucleotides in length, and the antisense strand is 19-26 nucleotides in length. The length ratio of the sense strand to the antisense strand of the RNAi agent provided by the present disclosure may be 19/19, 19/20, 19/21, 19/22, 19/23, 19/24, 19/25, 19/26, 20/19, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25, or 23/26. In some embodiments, the length ratio of the sense strand to the antisense strand of the RNAi agent is 19/21, 21/23, or 23/25. In some embodiments, the length ratio of the sense strand to the antisense strand of the RNAi agent is 19/21.

In some embodiments, the sense strand comprises a sequence of at least 15 contiguous nucleotides differing from any one of the nucleotide sequences of SEQ ID NO: 1 to SEQ ID NO: 11 and SEQ ID NO: 23 to SEQ ID NO: 65 by no more than 2 nucleotides; in some embodiments, by no more than one nucleotide; and in some embodiments, by one nucleotide.

In some embodiments, the antisense strand comprises a sequence of at least 15 contiguous nucleotides differing from any one of the nucleotide sequences of SEQ ID NO: 12 to SEQ ID NO: 22 and SEQ ID NO: 66 to SEQ ID NO: 108 by no more than 2 nucleotides; in some embodiments, by no more than one nucleotide; and in some embodiments, by one nucleotide.

In some embodiments, the sense strand comprises at least 15 contiguous nucleotides of any one of the nucleotide sequences of SEQ ID NO: 1 to SEQ ID NO: 11 and SEQ ID NO: 23 to SEQ ID NO: 65. In some embodiments, the sense strand comprises at least 16 contiguous nucleotides of any one of the nucleotide sequences of SEQ ID NO: 1 to SEQ ID NO: 11 and SEQ ID NO: 23 to SEQ ID NO: 65. In some embodiments, the sense strand comprises at least 17 contiguous nucleotides of any one of the nucleotide sequences of SEQ ID NO: 1 to SEQ ID NO: 11 and SEQ ID NO: 23 to SEQ ID NO: 65. In some embodiments, the sense strand comprises at least 19 contiguous nucleotides of any one of the nucleotide sequences of SEQ ID NO: 1 to SEQ ID NO: 11 and SEQ ID NO: 23 to SEQ ID NO: 65.

In some embodiments, the antisense strand comprises at least 15 contiguous nucleotides of any one of the nucleotide sequences of SEQ ID NO: 12 to SEQ ID NO: 22 and SEQ ID NO: 66 to SEQ ID NO: 108. In some embodiments, the antisense strand comprises at least 17 contiguous nucleotides of any one of the nucleotide sequences of SEQ ID NO: 12 to SEQ ID NO: 22 and SEQ ID NO: 66 to SEQ ID NO: 108. In some embodiments, the antisense strand comprises at least 19 contiguous nucleotides of any one of the nucleotide sequences of SEQ ID NO: 12 to SEQ ID NO: 22 and SEQ ID NO: 66 to SEQ ID NO: 108. In some embodiments, the antisense strand comprises at least 20 contiguous nucleotides of any one of the nucleotide sequences of SEQ ID NO: 12 to SEQ ID NO: 22 and SEQ ID NO: 66 to SEQ ID NO: 108. In some embodiments, the antisense strand comprises at least 21 contiguous nucleotides of any one of the nucleotide sequences of SEQ ID NO: 12 to SEQ ID NO: 22 and SEQ ID NO: 66 to SEQ ID NO: 108.

In some embodiments, the sense strand comprises or is selected from the group consisting of any one of the following nucleotide sequences: SEQ ID NO: 1 to SEQ ID NO: 11 and SEQ ID NO: 23 to SEQ ID NO: 65.

In some embodiments, the antisense strand comprises or is selected from the group consisting of any one of the following nucleotide sequences: SEQ ID NO: 12 to SEQ ID NO: 22 and SEQ ID NO: 66 to SEQ ID NO: 108.

In some embodiments, the RNAi agent comprises or consists of any one of the following combinations of sense and antisense strands:
combination 1): a sense strand set forth in SEQ ID NO: 1 and an antisense strand set forth in SEQ ID NO: 12;
or a sense strand set forth in SEQ ID NO: 2 and an antisense strand set forth in SEQ ID NO: 13;
combination 2): a sense strand set forth in SEQ ID NO: 3 and an antisense strand set forth in SEQ ID NO: 14;
or a sense strand set forth in SEQ ID NO: 4 and an antisense strand set forth in SEQ ID NO: 15;
combination 3): a sense strand set forth in SEQ ID NO: 5 and an antisense strand set forth in SEQ ID NO: 16;
combination 4): a sense strand set forth in SEQ ID NO: 6 and an antisense strand set forth in SEQ ID NO: 17;
or a sense strand set forth in SEQ ID NO: 7 and an antisense strand set forth in SEQ ID NO: 18;
combination 6): a sense strand set forth in SEQ ID NO: 8 and an antisense strand set forth in SEQ ID NO: 19;
or a sense strand set forth in SEQ ID NO: 9 and an antisense strand set forth in SEQ ID NO: 20; and
combination 7): a sense strand set forth in SEQ ID NO: 10 and an antisense strand set forth in SEQ ID NO: 21;
or a sense strand set forth in SEQ ID NO: 11 and an antisense strand set forth in SEQ ID NO: 22.

In some embodiments, at least one nucleotide in the sense strand and/or the antisense strand is a modified nucleotide. In some embodiments, all nucleotides are modified nucleotides.

In some embodiments, three contiguous nucleotides in the sense strand of the RNAi agent are 2'-fluoro-modified nucleotides. In some embodiments, in the sense strand of the RNAi agent, in the direction from the 5' end to the 3' end, the three contiguous nucleotides at positions 7-9 of the 5' end are 2'-fluoro-modified nucleotides. In some embodiments, the three contiguous nucleotides at positions 7-9 of the 5' end of the sense strand of the RNAi agent are 2'-fluoro-modified nucleotides, and the nucleotides at the remaining positions of the sense strand are all 2'-methoxy-modified nucleotides.

In some embodiments, in the direction from the 5' end to the 3' end, each of the nucleotides at positions 2, 6, 12, 14, and 16 of the antisense strand is independently a 2'-fluoro-modified nucleotide. In some embodiments, in the direction from the 5' end to the 3' end, each of the nucleotides at positions 2, 6, 12, 14, and 16 of the antisense strand is independently a 2'-fluoro-modified nucleotide, or the nucleotide at position 2, 4, 6, 10, 12, 14, 16, or 18 of the antisense strand is independently a 2'-fluoro-modified nucleotide, and the nucleotides at the remaining positions of the antisense strand are all 2'-methoxy-modified nucleotides.

In some embodiments, the 5' end of the antisense strand comprises one phosphorus-containing group, and the phosphorus-containing group is a 5'-vinylphosphodiester group (5'-VP). When the 5'-end phosphorus-containing group is a 5'-vinylphosphodiester group (5'-VP), the 5'-VP may be a 5'-*E*-VP isomer (i.e., a trans-vinylphosphodiester group), a 5'-*Z*-VP isomer (i.e., a cis-vinylphosphodiester group), or a mixture thereof. In some embodiments, the 5' end of the antisense strand comprises one phosphorus-containing group, and the phosphorus-containing group is a 5'-*E*-VP isomer (i.e., a trans-vinylphosphodiester group).

In some embodiments, at least one phosphodiester group in the sense strand and/or the antisense strand of the RNAi agent of the present disclosure is a phosphodiester group with a modification group. The modification group causes the RNAi agent to have increased stability in a biological sample or environment. In some embodiments, the phosphodiester group with a modification group is a phosphorothioate diester group.

In some embodiments, the phosphorothioate diester group is present in at least one position selected from the group consisting of the following positions:
between the 1st and 2nd nucleotides of the 5' end of the sense strand;
between the 2nd and 3rd nucleotides of the 5' end of the sense strand;
between the 1st and 2nd nucleotides of the 3' end of the sense strand;
between the 2nd and 3rd nucleotides of the 3' end of the sense strand;
between the 1st and 2nd nucleotides of the 5' end of the antisense strand;
between the 2nd and 3rd nucleotides of the 5' end of the antisense strand;
between the 1st and 2nd nucleotides of the 3' end of the antisense strand; and
between the 2nd and 3rd nucleotides of the 3' end of the antisense strand.

In some embodiments, the sense strand and/or the antisense strand comprise(s) a plurality of phosphodiester groups with modification groups, e.g., 1, 2, 3, 4, 5, or 6 phosphorothioate diester groups.

In some embodiments, the sense strand of the RNAi agent of the present disclosure comprises 2 phosphorothioate diester groups present in the following positions:
between the 1st and 2nd nucleotides of the 5' end of the sense strand; and
between the 2nd and 3rd nucleotides of the 5' end of the sense strand; and
the antisense strand of the RNAi agent of the present disclosure comprises 4 phosphorothioate diester groups present in the following positions:
   between the 1st and 2nd nucleotides of the 5' end of the antisense strand;
   between the 2nd and 3rd nucleotides of the 5' end of the antisense strand;
   between the 1st and 2nd nucleotides of the 3' end of the antisense strand; and
   between the 2nd and 3rd nucleotides of the 3' end of the antisense strand.

In some embodiments, the sense strand and the antisense strand each comprise 4 phosphorothioate diester groups present in the following positions:
between the 1st and 2nd nucleotides of the 5' end of the sense strand;
between the 2nd and 3rd nucleotides of the 5' end of the sense strand;
between the 1st and 2nd nucleotides of the 3' end of the sense strand;
between the 2nd and 3rd nucleotides of the 3' end of the sense strand;
between the 1st and 2nd nucleotides of the 5' end of the antisense strand;
between the 2nd and 3rd nucleotides of the 5' end of the antisense strand;
between the 1st and 2nd nucleotides of the 3' end of the antisense strand; and
between the 2nd and 3rd nucleotides of the 3' end of the antisense strand.

In some embodiments, the RNAi agent is linked to one or more lipophilic groups.

In some embodiments, the one or more lipophilic groups are linked to one or more nucleotides in the sense strand of the RNAi agent; in some embodiments, the one or more lipophilic groups are linked to one or more nucleotides in the antisense strand of the RNAi agent; in some embodiments, the one or more lipophilic groups are linked to multiple nucleotides in the sense strand and the antisense strand of the RNAi agent.

In some embodiments, the one or more lipophilic groups are linked to one or more bases; in some embodiments, the one or more lipophilic groups are linked to one or more sugar rings; in some embodiments, the one or more lipophilic groups are linked to one or more internucleoside linking groups.

In some embodiments, the one or more lipophilic groups are linked to the nucleotides at positions 2-8 in the middle of the sense strand; in some embodiments, the one or more lipophilic groups are linked to the nucleotides at positions 2-8 in the middle of the antisense strand.

In some embodiments, the one or more lipophilic groups are linked to at least one of the following positions:
the 1st nucleotide of the 5' end of the sense strand; the 1st nucleotide of the 5' end of the antisense strand; the 1st nucleotide of the 3' end of the sense strand; the 1st nucleotide of the 3' end of the antisense strand; and the 6th nucleotide of the 5' end of the sense strand.

In some embodiments, the one or more lipophilic groups are linked to the 6th nucleotide of the 5' end of the sense strand.

In some embodiments, the lipophilic group is linked to the 6th nucleotide in the direction from the 5' end to the 3' end of the sense strand. In some embodiments, the lipophilic group is linked to the 1st nucleotide of the 5' end of the sense strand.

In some embodiments, the lipophilic group comprises a saturated or unsaturated C₄-₃₀ hydrocarbon chain, and optionally a functional group selected from the group consisting of halogen, alkoxy, hydroxy, amine, carboxylic acid, sulfonate, phosphate, thiol, azide, and alkyne. In some embodiments, the lipophilic group comprises a saturated or unsaturated C₆₋₁₈ hydrocarbon chain; in some embodiments, the lipophilic group comprises a saturated or unsaturated C₁₆ hydrocarbon chain. In some embodiments, the saturated or unsaturated C₁₆ hydrocarbon chain is linked to the 1st nucleotide of the 5' end of the sense strand.

In some embodiments, the lipophilic group is linked to the 2' position of the sugar ring of the 1st or 6th nucleoside of the 5' end of the sense strand. In some embodiments, the aforementioned nucleoside to which a lipophilic group is linked is a nucleoside with -On-hexadecyl linked to the 2' position of the sugar ring or a uracil nucleoside with -CH₂On-hexadecyl linked to the 2' position of the sugar ring, and the nucleosides have structures represented by formulas (2) and (3), respectively: 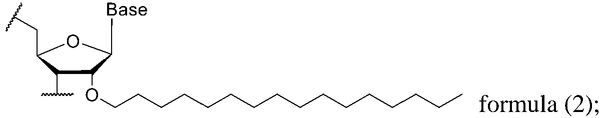 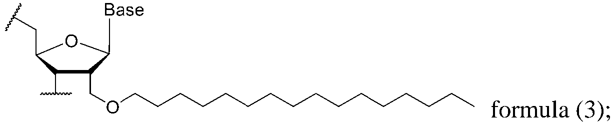 in formulas (2) and (3), Base represents a base.

The present disclosure further provides an RNAi agent comprising any one of the RNAi agents of the present disclosure and a targeting ligand linked to the RNAi agent.

In some embodiments, the RNAi agent and the targeting ligand are linked covalently or non-covalently.

In some embodiments, the targeting ligand targets the liver; in some embodiments, the targeting ligand binds to asialoglycoprotein receptor (ASGPR); in some embodiments, the targeting ligand comprises a galactose cluster or a galactose derivative cluster, wherein the galactose derivative is selected from the group consisting of N-acetyl-galactosamine, N-trifluoroacetylgalactosamine, N-propionylgalactosamine, N-n-butyrylgalactosamine, and N-isobutyrylgalactosamine.

In some embodiments, the targeting ligand is linked to the 3' end of the sense strand of the RNAi agent.

In some embodiments, the targeting ligand is linked to an end of the RNAi agent by a phosphodiester group, a phosphorothioate diester group, or a phosphonic acid group; in some embodiments, the targeting ligand is linked to an end of the RNAi agent by a phosphodiester group.

In some embodiments, the targeting ligand is indirectly linked to an end of the RNAi agent by a phosphodiester group, a phosphorothioate diester group, or a phosphonic acid group; in some embodiments, the targeting ligand is indirectly linked to an end of the RNAi agent by a phosphodiester group.

In some embodiments, the targeting ligand is directly linked to an end of the RNAi agent by a phosphodiester group, a phosphorothioate diester group, or a phosphonic acid group; in some embodiments, the targeting ligand is directly linked to an end of the RNAi agent by a phosphodiester group.

In some embodiments, the targeting ligand is directly linked to the 3' end of the sense strand of the RNAi agent by a phosphodiester group or a phosphorothioate diester group; in some embodiments, the targeting ligand is directly linked to the 3' end of the sense strand of the RNAi agent by a phosphodiester group.

In some embodiments, the targeting moiety of the targeting ligand consists of one or more targeting groups or targeting moieties, and the targeting ligand assists in directing the delivery of a therapeutic agent linked thereto to the desired target location. In some cases, the targeting moiety may bind to a cell or cellular receptor and initiate endocytosis to promote entry of the therapeutic agent into the cell. The targeting moiety may comprise a compound with affinity for a cellular receptor or a cell surface molecule or an antibody. Various targeting ligands comprising targeting moieties may be linked to therapeutic agents and other compounds to target the agents at cells and specific cellular receptors. In some embodiments, the types of the targeting moieties include carbohydrates, cholesterol and cholesterol groups, or steroids. Targeting moieties capable of binding to cellular receptors include saccharides such as galactose, galactose derivatives (e.g., N-acetyl-galactosamine, N-trifluoroacetylgalactosamine, N-propionylgalactosamine, N-n-butyrylgalactosamine, and N-isobutyrylgalactosamine), mannose, and mannose derivatives).

Targeting moieties that bind to asialoglycoprotein receptors (ASGPRs) are known to be particularly used for directing the delivery of oligomeric compounds to the liver. Asialoglycoprotein receptors are extensively expressed on liver cells (hepatocytes). Cellular receptor targeting moieties targeting ASGPRs include galactose and galactose derivatives. Specifically, clusters of galactose derivatives, including clusters consisting of 2, 3, 4, or more than 4 N-acetyl-galactosamine (GalNAc or NAG) molecules, can promote the uptake of certain compounds in hepatocytes. The GalNAc cluster coupled to the oligomeric compound is used for directing the composition to the liver, where the N-acetyl-galactosamine saccharide can bind to the asialoglycoprotein receptors on the liver cell surface. It is believed that the binding to the asialoglycoprotein receptors will initiate receptor-mediated endocytosis, thereby promoting entry of the compound into the interior of the cell.

In some embodiments, the targeting ligand may comprise 2, 3, 4, or more than 4 targeting moieties. In some embodiments, the targeting ligand disclosed herein may comprise 1, 2, 3, 4, or more than 4 targeting moieties linked to a branching group by L₂.

In some embodiments, each targeting moiety comprises a galactosamine derivative that is N-acetyl-galactosamine. Other sugars that may be used as targeting moieties and have affinity for asialoglycoprotein receptors may be selected from the group consisting of galactose, galactosamine, N-formyl-galactosamine, N-acetyl-galactosamine, N-propionyl-galactosamine, N-n-butyryl-galactosamine, N-isobutyryl-galactosamine, etc. In some embodiments, the targeting ligand in the present disclosure comprises N-acetylgalactosamine as a targeting moiety,

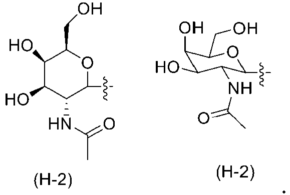

In some embodiments, the targeting ligand comprises three terminal galactosamines or galactosamine derivatives (such as N-acetyl-galactosamine), and each has affinity for asialoglycoprotein receptors. In some embodiments, the targeting ligand comprises three terminal N-acetyl-galactosamines (GalNAc or NAG) as targeting moieties.

In some embodiments, the targeting ligand comprises four terminal galactosamines or galactosamine derivatives (such as N-acetyl-galactosamine), and each has affinity for asialoglycoprotein receptors. In some embodiments, the targeting ligand comprises four terminal N-acetyl-galactosamines (GalNAc or NAG) as targeting moieties.

Terms commonly used in the art when referring to three terminal N-acetyl-galactosamines include tri-antennary,
tri-valent, and trimer.

Terms commonly used in the art when referring to four terminal N-acetyl-galactosamines include tetra-antennary,
tetra-valent, and tetramer.

In some embodiments, the targeting ligand provided by the present disclosure is a compound as shown below or a pharmaceutically acceptable salt thereof: 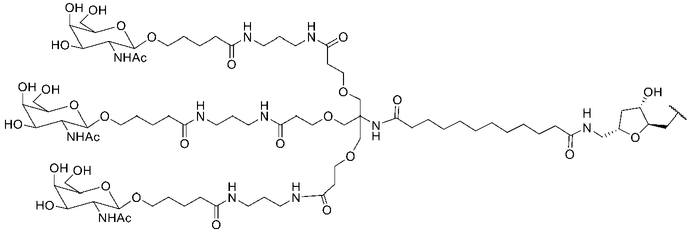 or

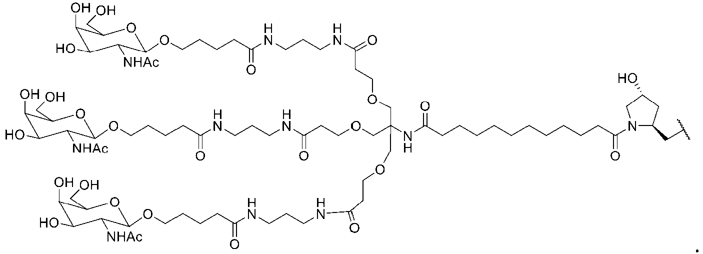

In some embodiments, the N-acetyl-galactosamine moiety in the above targeting ligands may be replaced with N-trifluoroacetylgalactosamine, N-propionylgalactosamine, N-n-butyrylgalactosamine, or N-isobutyrylgalactosamine.

In some embodiments, the sense strand and the antisense strand of the RNAi agent of the present disclosure are provided, wherein the sense strand comprises the nucleotide sequence set forth in any one of SEQ ID NO: 109 to SEQ ID NO: 125 and SEQ ID NO: 143 to SEQ ID NO: 185;
and/or the antisense strand comprises the nucleotide sequence set forth in any one of SEQ ID NO: 126 to SEQ ID NO: 142 and SEQ ID NO: 186 to SEQ ID NO: 228.

In some more specific embodiments, the RNAi agent comprises or consists of any one of the following combinations of sense and antisense strands:
a sense strand set forth in SEQ ID NO: 109 and an antisense strand set forth in SEQ ID NO: 126;
a sense strand set forth in SEQ ID NO: 110 and an antisense strand set forth in SEQ ID NO: 127;
a sense strand set forth in SEQ ID NO: 111 and an antisense strand set forth in SEQ ID NO: 128;
a sense strand set forth in SEQ ID NO: 112 and an antisense strand set forth in SEQ ID NO: 129;
a sense strand set forth in SEQ ID NO: 113 and an antisense strand set forth in SEQ ID NO: 130;
a sense strand set forth in SEQ ID NO: 114 and an antisense strand set forth in SEQ ID NO: 131;
a sense strand set forth in SEQ ID NO: 115 and an antisense strand set forth in SEQ ID NO: 132;
a sense strand set forth in SEQ ID NO: 116 and an antisense strand set forth in SEQ ID NO: 133;
a sense strand set forth in SEQ ID NO: 117 and an antisense strand set forth in SEQ ID NO: 134;
a sense strand set forth in SEQ ID NO: 118 and an antisense strand set forth in SEQ ID NO: 135;
a sense strand set forth in SEQ ID NO: 119 and an antisense strand set forth in SEQ ID NO: 136;
a sense strand set forth in SEQ ID NO: 120 and an antisense strand set forth in SEQ ID NO: 137;
a sense strand set forth in SEQ ID NO: 121 and an antisense strand set forth in SEQ ID NO: 138;
a sense strand set forth in SEQ ID NO: 122 and an antisense strand set forth in SEQ ID NO: 139;
a sense strand set forth in SEQ ID NO: 123 and an antisense strand set forth in SEQ ID NO: 140;
a sense strand set forth in SEQ ID NO: 124 and an antisense strand set forth in SEQ ID NO: 141; and
a sense strand set forth in SEQ ID NO: 125 and an antisense strand set forth in SEQ ID NO: 142.

In another aspect, the present disclosure provides a pharmaceutical composition comprising the RNAi agent described in the present disclosure, and one or more pharmaceutically acceptable excipients, such as vehicles, carriers, diluents, and/or delivery polymers. The term "pharmaceutical composition" refers to a mixture containing one or more of the compounds or the physiologically and pharmaceutically acceptable salts or pro-drugs thereof described herein, and other chemical components, as well as other components such as physiologically and pharmaceutically acceptable carriers and excipients. The pharmaceutical composition is intended to promote administration to an organism and facilitate the absorption of the active ingredient so that it can exert its biological activity.

Various drug delivery systems are known and can be used for the RNAi agent of the present disclosure, e.g., encapsulation in liposomes, microparticles, microcapsules, recombinant cells capable of expressing the RNAi agent, receptor-mediated endocytosis, and construction of a nucleic acid as part of a retroviral vector or other vectors.

In some embodiments, the pharmaceutical composition may further comprise a pharmaceutically acceptable auxiliary material and/or adjuvant, and the auxiliary material may be one or more of the various formulations or compounds conventionally used in the art. For example, the pharmaceutically acceptable auxiliary material may include at least one of a pH buffer, a protective agent, and an osmotic pressure regulator.

In some embodiments, a unit dose of the pharmaceutical composition is 0.001 mg-1000 mg.

In some embodiments, the effective amount or effective dose of the RNAi agent and/or the pharmaceutical composition is about 0.001 mg/kg body weight to about 200 mg/kg body weight, about 0.01 mg/kg body weight to about 100 mg/kg body weight, or about 0.5 mg/kg body weight to about 50 mg/kg body weight.

In certain embodiments, the pharmaceutical composition comprises 0.01-99.99% of the aforementioned compound or a pharmaceutically acceptable salt thereof or an isotopically substituted form thereof based on the total weight of the composition. In certain embodiments, the pharmaceutical composition comprises 0.1-99.9% of the aforementioned compound or the pharmaceutically acceptable salt thereof or the isotopically substituted form thereof. In certain embodiments, the pharmaceutical composition comprises 0.5%-99.5% of the aforementioned compound or the pharmaceutically acceptable salt thereof or the isotopically substituted form thereof. In certain embodiments, the pharmaceutical composition comprises 1%-99% of the aforementioned compound or the pharmaceutically acceptable salt thereof or the isotopically substituted form thereof. In certain embodiments, the pharmaceutical composition comprises 2%-98% of the aforementioned compound or the pharmaceutically acceptable salt thereof or the isotopically substituted form thereof.

The pharmaceutical composition may be conveniently presented in a unit dosage form. In general, the pharmaceutical composition may be formulated into any suitable dosage form, such as, but not limited to, injections, tablets, capsules, gels, etc. The pharmaceutical composition includes, but is not limited to, a solution, an emulsion, and a liposome-containing formulation.

In certain embodiments, the pharmaceutical composition comprises 0.01%-99.99% of a pharmaceutically acceptable excipient based on the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

In another aspect, the present disclosure further provides a cell comprising the RNAi agent of the present disclosure.

In another aspect, the present disclosure further provides a kit comprising the RNAi agent or the pharmaceutical composition of the present disclosure.

In another aspect, the present disclosure further provides a method for silencing the APP gene or mRNA expressed by the APP gene in a cell, and the method comprises a step of introducing into the cell the RNAi agent and/or the pharmaceutical composition according to the present disclosure. The present disclosure further provides a method for silencing the APP gene or mRNA expressed by the APP gene in a cell *in vivo* or *in vitro*, and the method comprises a step of introducing into the cell the RNAi agent and/or the pharmaceutical composition according to the present disclosure. The present disclosure further provides a method for reducing APP gene expression, and the method comprises administering to a subject the RNAi agent and/or the pharmaceutical composition of the present disclosure.

In another aspect, the present disclosure provides use of the RNAi agent or the pharmaceutical composition described in the present disclosure in the manufacture of a medicament for treating and/or preventing a disease in a subject. In some embodiments, the disease is a disease associated with the APP gene. In some embodiments, the disease is a hepatic disease or a central neurodegenerative disease. In some embodiments, the disease is selected from the group consisting of sporadic Alzheimer's disease, hereditary/familial Alzheimer's disease, Down syndrome, frontotemporal dementia, cerebral amyloid angiopathy, and Alzheimer's disease and cerebral amyloid angiopathy in patients with Down syndrome.

In another aspect, the present disclosure further provides a method for treating and/or preventing a disease, comprising administering to a subject the RNAi agent and/or the pharmaceutical composition of the present disclosure. In some embodiments, the disease is a disease associated with the APP gene. In some embodiments, the disease is a hepatic disease or a central neurodegenerative disease. In some embodiments, the disease is selected from the group consisting of sporadic Alzheimer's disease, hereditary/familial Alzheimer's disease, Down syndrome, frontotemporal dementia, cerebral amyloid angiopathy, and Alzheimer's disease and cerebral amyloid angiopathy in patients with Down syndrome.

The present disclosure provides a method for treating and/or preventing a disease associated with APP gene expression in a subject, comprising administering to the subject an effective amount or effective dose of the RNAi agent or the pharmaceutical composition described in the present disclosure, wherein the RNAi agent or the pharmaceutical composition is capable of inhibiting APP gene expression and/or replication.

The present disclosure provides use of the RNAi agent and/or the pharmaceutical composition of the present disclosure in the manufacture of a medicament for treating and/or preventing a disease associated with APP gene expression.

The present disclosure provides use of the RNAi agent and/or the pharmaceutical composition of the present disclosure in the manufacture of a medicament for inhibiting APP expression.

The present disclosure provides a method for inhibiting APP expression, comprising administering to a subject an effective amount or effective dose of the RNAi agent and/or the pharmaceutical composition of the present disclosure.

The present disclosure provides a method for delivering an RNAi agent inhibiting APP expression and/or replication *in vivo*, and the method comprises administering to a subject the RNAi agent and/or the pharmaceutical composition of the present disclosure.

In another aspect, the present disclosure provides a method for delivering an RNAi agent inhibiting APP expression and/or replication *in vivo*, and the method comprises administering to a subject the RNAi agent and/or the pharmaceutical composition of the present disclosure.

In some embodiments, the subject has been previously identified as having pathological up-regulation of the target gene in the targeted cell or tissue.

The subject described in the present disclosure refers to a subject having (or suspected to have or susceptible to) a disease or disorder that would benefit from reduction or inhibition of target mRNA expression.

Delivery may be accomplished by topical administration (e.g., direct injection, implantation, or topical application), systemic administration, or through subcutaneous, intravenous, intraperitoneal, or parenteral routes, including intracranial (e.g., intraventricular, intraparenchymal, and intrathecal), intramuscular, transdermal, airway (aerosol), nasal, oral, rectal, or topical (including buccal and sublingual) administration. In an optional embodiment, the pharmaceutical composition provided by the present disclosure may be administered by injection, e.g., intravenous, intramuscular, intradermal, subcutaneous, intraduodenal, or intraperitoneal injection.

In an optional embodiment, after the targeting ligand is linked to the RNAi agent to form a conjugate, the conjugate can be packaged in a kit.

In some embodiments, when the RNAi agent or the pharmaceutical composition of the present disclosure is in contact with a target gene-expressing cell, the RNAi agent or the pharmaceutical composition of the present disclosure inhibits the expression of the target gene by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the RNAi agent or the pharmaceutical composition of the present disclosure is in contact with a target gene-expressing cell, the RNAi agent or the pharmaceutical composition of the present disclosure results in a remaining expression percentage of the target gene mRNA of no greater than 99%, no greater than 95%, no greater than 90%, no greater than 85%, no greater than 80%, no greater than 75%, no greater than 70%, no greater than 65%, no greater than 60%, no greater than 55%, no greater than 50%, no greater than 45%, no greater than 40%, no greater than 35%, no greater than 30%, no greater than 25%, no greater than 20%, no greater than 15%, or no greater than 10%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the RNAi agent or the pharmaceutical composition of the present disclosure is in contact with a target gene-expressing cell, the RNAi agent, while retaining on-target activity, reduces off-target activity by at least 20%, at least 25%, at least 30%, at least 35%, at least 40%, at least 45%, at least 50%, at least 55%, at least 60%, at least 65%, at least 70%, or at least 75%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the RNAi agent or the pharmaceutical composition of the present disclosure is in contact with a target gene-expressing cell, the RNAi agent, while reducing on-target activity by at most 20%, at most 19%, at most 15%, at most 10%, at most 5%, or more than 1%, reduces off-target activity by at least 20%, at least 25%, at least 30%, at least 35%, at least 40%, at least 45%, at least 50%, at least 55%, at least 60%, at least 65%, at least 70%, or at least 75%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the RNAi agent or the pharmaceutical composition of the present disclosure is in contact with a target gene-expressing cell, the RNAi agent, while increasing on-target activity by at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, or at least 80%, reduces off-target activity by at least 20%, at least 25%, at least 30%, at least 35%, at least 40%, at least 45%, at least 50%, at least 55%, at least 60%, at least 65%, at least 70%, or at least 75%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, administration is performed through administration modes including intramuscular, intrabronchial, intrapleural, intraperitoneal, intraarterial, lymphatic, intravenous, subcutaneous, cerebrospinal, or combinations thereof.

The present disclosure further provides a method for preparing an RNAi agent or a pharmaceutical composition, comprising: synthesizing the RNAi agent or the pharmaceutical composition described in the present disclosure.

The present disclosure further provides an RNAi agent, wherein one or more bases U, e.g., 1, 2, 3, 3, 5, 6, 7, 8, 9, or 10 bases U, of any RNAi agent of the present disclosure are replaced with bases T.

The pharmaceutically acceptable salts of the compounds described in the present disclosure are selected from the group consisting of inorganic salts and organic salts. The compounds described in the present disclosure can react with acidic or basic substances to form corresponding salts.

In another aspect, where the configuration is not specified, the compounds of the present disclosure may have particular geometric or stereoisomeric forms. The present disclosure contemplates all such compounds, including cis and trans isomers, (-)- and (+)-enantiomers, (R)- and (S)-enantiomers, diastereomers, (D)-isomer, (L)-isomer, and racemic mixtures and other mixtures thereof, such as enantiomerically or diastereomerically enriched mixtures, all of which fall within the scope of the present disclosure. Additional asymmetric carbon atoms may be present in substituents such as an alkyl group. All such isomers and mixtures thereof fall within the scope of the present disclosure.

In addition, when the configuration is not specified, the compounds and intermediates of the present disclosure can also be present in different tautomeric forms, and all such forms fall within the scope of the present disclosure. The term "tautomer" or "tautomeric form" refers to structural isomers of different energies that can interconvert via a low-energy barrier. For example, proton tautomers (also known as proton transfer tautomers) include interconversion via proton migration, such as keto-enol, imine-enamine, and lactam-lactim isomerization. All tautomeric forms fall within the scope of the present disclosure. The names of the compounds do not exclude any tautomers.

The compounds of the present disclosure may be asymmetric, for example, having one or more stereoisomers. Unless otherwise specified, all stereoisomers are included, for example, enantiomers and diastereomers. The compounds of the present disclosure containing asymmetric carbon atoms may be separated in an optically active pure form or in a racemic form. The optically active pure form may be isolated from a racemic mixture or synthesized using chiral starting materials or chiral reagents.

Optically active (R)- and (S)-isomers, and D- and L-isomers may be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated, and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., carbamate formation from amines). The present disclosure also includes some isotopically labeled compounds of the present disclosure that are identical to those recited herein but have one or more atoms replaced with an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Unless otherwise specified, when a position is specifically designated as deuterium (D), the position shall be understood to be deuterium having an abundance that is at least 1000 times greater than the natural abundance of deuterium (which is 0.015%) (i.e., at least 10% deuterium incorporation). The compounds in examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure also includes various deuterated forms of the compounds of formula I and formula II. Each available hydrogen atom linked to a carbon atom may be independently replaced with a deuterium atom. Those skilled in the art can synthesize the deuterated forms of the compounds of formula I and formula II with reference to the relevant literature. Commercially available deuterated starting materials can be used in preparing the deuterated forms of the compounds of formula I and formula II, or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, and the like.

Where the configurations are not specified, in the chemical structures of the compounds of the present disclosure, the bond " 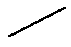 " indicates an unspecified configuration; that is, if chiral isomers exist in the chemical structures, the bond " 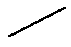 " may be " 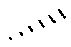 " or " " 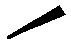 ", or includes both the configurations " 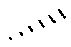 " and " 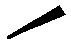 " simultaneously. Although all of the structural formulas of the present disclosure are drawn as certain isomeric forms for the sake of simplicity, the present disclosure may include all isomers, such as tautomers, rotamers, geometric isomers, diastereomers, racemates, and enantiomers. In the chemical structures of the compounds of the present disclosure, the bond " 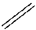 " does not specify a configuration; that is, the configuration of the bond " 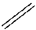 " may be an E configuration or a Z configuration, or includes both the E configuration and the Z configuration simultaneously.

WO2022028462A1, WO2023274395A1, and WO2023208023A1 are incorporated in the present disclosure by reference in their entirety.

### Terms and Definitions

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains. As used herein, "RNAi agent" (also referred to as "RNAi trigger agent") means a pharmaceutical composition comprising an RNA or RNA-like (e.g., chemically modified RNA) oligonucleotide molecule that is capable of degrading or inhibiting (e.g., degrading or inhibiting under appropriate conditions) translation of a messenger RNA (mRNA) transcript of a target mRNA in a sequence-specific manner. The RNAi agent used herein may act through an RNA interference mechanism (i.e., inducing RNA interference through interaction with the RNA interference pathway machinery (RNA-induced silencing complex or RISC) of mammalian cells) or through any alternative mechanism or pathway. Although the term RNAi agent as used herein is believed to act primarily through an RNA interference mechanism, the disclosed RNAi agent is not bound or limited by any particular pathway or mechanism of action. The RNAi agent disclosed herein consists of a sense strand and an antisense strand, and includes, but is not limited to, short (or small) interfering RNAs (siRNAs). The antisense strand of the RNAi agent described herein is at least partially complementary to the targeted mRNA. The RNAi agent may comprise one or more modified nucleotides and/or one or more non-phosphodiester linkages.

Unless otherwise specified, in the context of the present disclosure, the terms "amyloid precursor protein", "amyloid β precursor protein", "APP", "ABPP", "amyloid beta precursor protein", and "β-amyloid precursor protein" are used interchangeably in the present disclosure. APP includes, but is not limited to, human APP, cynomolgus monkey APP, mouse APP, and rat APP, and their amino acids, complete coding sequences, and mRNA sequences are readily accessible using publicly available databases, e.g., GenBank, UniProt, OMIM, and the Macaca genome project website.

The term "APP" also refers to naturally occurring DNA sequence variations of the APP gene, such as a single nucleotide polymorphism (SNP) in the APP gene. Exemplary SNPs may be found in the dbSNP database.

The term "target sequence" refers to a contiguous portion of the nucleotide sequence of an mRNA molecule formed during the transcription of APP, including mRNA that is a product of RNA processing of a primary transcription product. In one embodiment, the target sequence is within the protein-coding region of APP.

The term "neurodegenerative disease associated with the APP gene" includes any disease or disorder associated with reduced APP expression and/or activity. Symptoms or hallmarks often include cognitive disorders, such as declines in memory and speech; behavioral and psychological symptoms, such as apathy and lack of motivation, gait disturbances, and seizures; and progressive dementia and abnormal amyloid deposition. Typical "neurodegenerative diseases associated with the APP gene" include sporadic Alzheimer's disease, hereditary/familial Alzheimer's disease, and Alzheimer's disease and cerebral amyloid angiopathy in patients with Down syndrome.

As used herein, in the context of RNA-mediated gene silencing, the sense strand (also referred to as SS or SS strand) refers to a strand comprising a sequence identical or substantially identical to a target mRNA sequence; the antisense strand (also referred to as AS or AS strand) refers to a strand comprising a sequence complementary to a target mRNA sequence.

In the context describing the sense strand of the RNAi agent described herein, the term "a sequence differing from any one of the nucleotide sequences of SEQ ID NO: 1 to SEQ ID NO: 11 by no more than 3 nucleotides and comprises at least 15 contiguous nucleotides" is intended to mean that the sense strand of the RNAi agent described herein comprises at least 15 contiguous nucleotides of any one of the sense strands of SEQ ID NO: 1 to SEQ ID NO: 11, or a sequence differing from at least 15 contiguous nucleotides of any one of the sense strands of SEQ ID NO: 1 to SEQ ID NO: 11 by no more than 3 nucleotides (optionally by no more than 2 nucleotides; optionally by one nucleotide).

In the present disclosure, the "5' region", "5' end", and "5' terminus" of the sense or antisense strand are used interchangeably. For example, the nucleotides at positions 2 to 8 of the 5' region of the antisense strand may also be replaced with the nucleotides at positions 2 to 8 of the 5' end of the antisense strand. Likewise, the "3' region", "3' terminus", and "3' end" of the sense or antisense strand are also used interchangeably.

Unless otherwise specified, in the context of the present disclosure, "G", "C", "A", "T", and "U" represent nucleosides, and comprise the bases of guanine, cytosine, adenine, thymidine, and uracil, respectively. It is well-known to those skilled in the art that the mutual replacement between bases T and U does not significantly affect the properties of the RNAi agent sequence. In the sequences of the present disclosure, U can be arbitrarily replaced with T, and the sequences after the replacement also fall within the protection scope of the present disclosure. In the sequences of the present disclosure, for the same nucleic acid strand, the direction from the 5' end to the 3' end is a direction from the left to the right; the lowercase letter m means that the nucleoside adjacent to the letter m on the left side is a 2'-methoxy-modified nucleoside; the lowercase letter f means that the nucleoside adjacent to the letter f on the left side is a 2'-fluoro-modified nucleoside; VP means that the 5' end of the nucleoside adjacent to the letters on the left/right side is a trans-vinylphosphodiester group (5'-*E*-VP); the lowercase letter s means that the two nucleosides adjacent to the letter s are linked by a phosphorothioate diester group; unless otherwise specified, two adjacent nucleosides are linked by a phosphodiester group. Unless otherwise specified, the "RNAi agent", "nucleotide", "compound", "chemical modification", "oligonucleotide", "double-stranded RNAi inhibitor molecule", "siRNA", "siRNA conjugate", "dsRNA", "nucleic acid", "RNAi", and "nucleoside" of the present disclosure can each independently be present in the form of a salt, a mixed salt, or a non-salt (e.g., a free acid or free base). When being present in the form of a salt or mixed salts, it may be a pharmaceutically acceptable salt. The term "pharmaceutically acceptable salt" includes pharmaceutically acceptable acid addition salts and pharmaceutically acceptable base addition salts. When it is present in the form of a salt, part of the groups may be ionized to form anions/cations; for example, phosphodiester groups and phosphorothioate diester groups may be present in the form of anions, and the structures in the form of a salt corresponding to the following structures also fall within the protection scope of the present disclosure. Unless otherwise specified, the 3' position of the first nucleotide at the 3' end of each strand is hydroxy; the 5' position of the first nucleotide at the 5' end of each strand is hydroxy.

The modifications and linking groups described above separately have the structures shown in the table below, where Base represents a base:

**Table 1**

| Structure | Name |
|---|---|
| 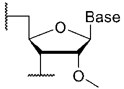 | 2'-Methoxy-modified nucleoside |
| 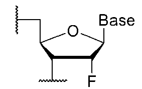 | 2'-Fluoro-modified nucleoside |
| 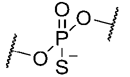 | Anion form of phosphorothioate diester group |
| 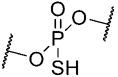 | Phosphorothioate diester group |
| 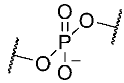 | Anion form of phosphodiester group |
| 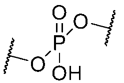 | Phosphodiester group |
| 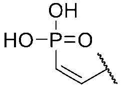 | Z-Vinylphosphoric acid group |
| | (Z-VP) |
| 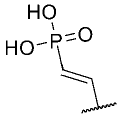 | E-Vinylphosphoric acid group |
| | (*E*-VP) |

The term "lipophilic group" or "lipophilic moiety" broadly refers to any compound or chemical moiety that has an affinity for lipids. One way to characterize the lipophilicity of a lipophilic moiety is by the octanol-water partition coefficient, logK_{ow}, where K_{ow} is the ratio of the concentration of a chemical substance in the octanol phase to its concentration in the aqueous phase in a biphasic system in equilibrium. In principle, a chemical substance is lipophilic when its logK_{ow} exceeds 0. Typically, a lipophilic moiety has a logK_{ow} of greater than 1, greater than 1.5, greater than 2, greater than 3, greater than 4, greater than 5, or greater than 10; for example, 6-aminohexanol has a logK_{ow} of about 0.7, and cholesteryl N-(hexan-6-ol)carbamate has a logK_{ow} of 10.7.

The lipophilicity of a molecule may vary relative to the functional groups it carries. For example, adding a hydroxy group or an amine group to an end of a lipophilic moiety may increase or decrease the partition coefficient (e.g., logK_{ow}) value of the lipophilic moiety. For example, a lipophilic moiety may be an aliphatic, cyclic such as alicyclic, or polycyclic such as polyalicyclic compounds, such as steroids (e.g., sterols) or linear or branched aliphatic hydrocarbons. A lipophilic moiety may generally contain a hydrocarbon chain, and the hydrocarbon chain may be cyclic or acyclic. The hydrocarbon chain may contain various substituents and/or one or more heteroatoms, such as oxygen or sulfur atoms. Such lipophilic aliphatic moieties include, but are not limited to, saturated or unsaturated C₄-C₃₀ hydrocarbons (e.g., C₁₀-C₃₀ hydrocarbons), saturated or unsaturated fatty acids, waxes (e.g., monohydric alcohol esters of fatty acids and fatty diamides), terpenes (e.g., C₁₀ terpenes, C₁₅ sesquiterpenes, C₂₀ diterpenes, C₃₀ triterpenes, and C₄₀ tetraterpenes), and other polyalicyclic hydrocarbons; for example, a lipophilic moiety may be an optionally substituted C₁₀₋₃₀ linear alkyl group; for example, a lipophilic moiety may be an optionally substituted C₁₄₋₂₄ linear alkyl group.

As used herein, the terms "complementary" and "reverse complementary" are used interchangeably and have the meaning well-known to those skilled in the art; that is, in a double-stranded nucleic acid molecule, the bases of one strand are paired with the bases of the other strand in a complementary manner. In DNA, the purine base adenine is always paired with the pyrimidine base thymine (or uracil in RNA), and the purine base guanine is always paired with the pyrimidine base cytosine. Each base pair comprises a purine and a pyrimidine. When adenines of one strand are always paired with thymines (or uracils) of another strand, and guanines are always paired with cytosines, the two strands are considered complementary to each other, and the sequences of the strands can be deduced from the sequences of their complementary strands. Accordingly, "mismatch" in the art means that in a double-stranded nucleic acid, the bases at the corresponding positions are not paired in a complementary manner.

As used herein, the term "inhibit" is used interchangeably with "decrease", "silence", "down-regulate", "repress", and other similar terms, and includes any level of inhibition. Inhibition can be assessed in terms of a decrease in the absolute or relative level of one or more of these variables relative to a control level. The control level can be any type of control level used in the art, such as a pre-dose baseline level or a level determined from an untreated or control (e.g., buffer-only control or inert agent control) treated subject, cell, or sample. For example, the remaining mRNA expression level can be used to characterize the degree of inhibition of target gene expression by the RNAi agent; for example, the remaining expression level of mRNA is not greater than 99%, not greater than 95%, not greater than 90%, not greater than 85%, not greater than 80%, not greater than 75%, not greater than 70%, not greater than 65%, not greater than 60%, not greater than 55%, not greater than 50%, not greater than 45%, not greater than 40%, not greater than 35%, not greater than 30%, not greater than 25%, not greater than 20%, not greater than 15%, or not greater than 10%. The inhibition rate of target gene expression can be measured using Dual-Glo^{®} Luciferase Assay System: the Firefly chemiluminescence value (Fir) and the Renilla chemiluminescence value (Ren) are each read, and the relative value Ratio = Ren/Fir is calculated; in the present disclosure, the ratio of the remaining mRNA expression level (or residual activity%) = Ratio (RNAi agent-treated group)/Ratio (RNAi agent-free control group), and the inhibition rate (%) = 100% - remaining mRNA expression level (%).

Unless otherwise specified, the "compound", "ligand", "nucleic acid-ligand conjugate", "nucleic acid", "conjugate", "chemical modification", "targeting ligand", "dsRNA", "siRNA", and "RNAi agent" of the present disclosure can each independently be present in the form of a salt, a mixed salt, or a non-salt (e.g., a free acid or free base). When being present in the form of a salt or mixed salts, it may be a pharmaceutically acceptable salt. In the context of the present disclosure, in some embodiments, "dsRNA" and "RNAi agent" are interchangeable with each other.

The term "pharmaceutically acceptable salt" includes pharmaceutically acceptable acid addition salts and pharmaceutically acceptable base addition salts.

"Pharmaceutically acceptable acid addition salt" refers to salts that are capable of retaining the biological effectiveness of free bases without having any undesirable effects and that are formed with inorganic or organic acids. Inorganic acid salts include, but are not limited to, hydrochlorides, hydrobromides, sulfates, nitrates, phosphates, etc.; organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, mesylates, benzenesulfonates, p-toluenesulfonates, alginates, ascorbates, salicylates, 4-aminosalicylates, napadisylates, etc. These salts can be prepared using methods known in the art.

"Pharmaceutically acceptable base addition salt" refers to salts that are capable of retaining the biological effectiveness of free acids without having any undesirable effects and that are formed with inorganic bases or organic bases. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. The inorganic salts in some embodiments are ammonium salts, sodium salts, potassium salts, calcium salts, and magnesium salts. Salts derived from organic bases include, but are not limited to, salts of the following: primary, secondary, and tertiary amines, substituted amines including naturally occurring substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, etc. The organic bases in some embodiments include isopropylamine, diethylamine, ethanolamine, trimethylamine, dicyclohexylamine, choline, and caffeine. These salts can be prepared using methods known in the art.

"Effective amount" or "effective dose" refers to the amount of a drug, a compound, or a pharmaceutical composition necessary to obtain any one or more beneficial or desired therapeutic results. For prophylactic use, the beneficial or desired results include elimination or reduction of risk, reduction of severity, or delay of the onset of a disorder, including the biochemistry, histology, and/or behavioral symptoms of the disorder, complications thereof, and intermediate pathological phenotypes that appear during the progression of the disorder. For therapeutic applications, the beneficial or desired results include clinical results, such as reducing the incidence of various disorders related to the target gene, target mRNA, or target protein of the present disclosure or ameliorating one or more symptoms of the disorders, reducing the dose of other agents required to treat the disorders, enhancing the therapeutic effect of another agent, and/or delaying the progression of disorders related to the target gene, target mRNA, or target protein of the present disclosure in patients.

As used herein, "patient", "subject", and "individual" are used interchangeably and include human or non-human animals, e.g., mammals, e.g., humans or monkeys.

The RNAi agent provided by the present disclosure can be obtained using a preparation method conventional in the art (e.g., solid-phase synthesis and liquid-phase synthesis). Solid-phase synthesis has been commercially available as a customization service. A modified nucleotide group can be introduced into the RNAi agent described in the present disclosure using a nucleoside monomer with a corresponding modification. Methods of preparing a nucleoside monomer with a corresponding modification and introducing a modified nucleotide group into an RNAi agent are also well known to those skilled in the art.

The term "chemical modification" or "modification" includes all changes made to a nucleotide by chemical means, such as the addition or removal of a chemical moiety, or the substitution of one chemical moiety for another.

The term "base" encompasses any known DNA and RNA bases and base analogs such as purines or pyrimidines, and also includes the natural compounds adenine, thymine, guanine, cytosine, uracil, inosine, and natural analogs.

The terms "blunt" and "blunt-ended" are used interchangeably and mean that there are no unpaired nucleotides or nucleotide analogs at a given end of an RNAi agent, i.e., no nucleotide overhangs. In most cases, an RNAi agent whose ends are both blunt-ended will be double-stranded over its entire length.

The terms "about" and "approximately" mean that a numerical value is within an acceptable margin of error for the specific value determined by those of ordinary skill in the art, and the numerical value depends in part on how the value is measured or determined (i.e., the limitations of the measurement system). For example, "about" may mean a standard deviation within 1 or greater than 1. Alternatively, "about" or "substantially comprise" may mean a range of at most 20%, e.g., a change of between 1% and 15%, between 1% and 10%, between 1% and 5%, between 0.5% and 5%, or between 0.5% and 1%. In the present disclosure, every instance where a number or numerical range is preceded by the term "about" also includes an embodiment of the given number. Unless otherwise specified, when a specific value is provided in the present application and claims, the meaning of "about" or "substantially comprise" should be assumed to be within an acceptable margin of error for that specific value.

Unless otherwise specified, "optionally" or "optional" means that the event or circumstance subsequently described may, but does not necessarily, occur, and this description includes instances where the event or circumstance occurs or does not occur. For example, "optionally, R₁ and R₂ are directly linked to form a ring" means that R₁ and R₂ being directly linked to form a ring may occur, but does not necessarily exist, and this description includes an instance where R₁ and R₂ are directly linked to form a ring and an instance where R₁ and R₂ do not form a ring.

In the chemical structural formulas of the present disclosure, " 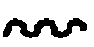 " or 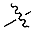 or 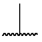 may be linked to any group or groups in accordance with the scope of the invention described herein.

The term "link", when referring to a relationship between two molecules, means that the two molecules are linked by a covalent bond or that the two molecules are associated via a non-covalent bond (e.g., a hydrogen bond or an ionic bond), and includes direct linkage and indirect linkage.

The term "directly linked" means that a first compound or group is linked to a second compound or group without any atom or group of atoms interposed between.

The term "indirectly linked" means that a first compound or group is linked to a second compound or group by an intermediate group, a compound, or a molecule (e.g., a linking group).

The term "substituted" means that any one or more hydrogen atoms on the specified atom (usually a carbon, oxygen, or nitrogen atom) are replaced with any group as defined herein, provided that the normal valency of the specified atom is not exceeded and that the substitution results in a stable compound. Non-limiting examples of substituents include C1-C6 alkyl, C2-C6 alkenyl, C2-C6 alkynyl, cyano, hydroxy, oxo, carboxyl, cycloalkyl, cycloalkenyl, heterocyclyl, heteroaryl, aryl, ketone, alkoxycarbonyl, aryloxycarbonyl, heteroaryloxycarbonyl, or halogen (e.g., F, Cl, Br, or I). When the substituent is a ketone or oxo (i.e., =O), two (2) hydrogens on the atom are replaced.

"Substituted with one or more..." refers to substitution with a single substituent or multiple substituents. In the case of substitution with multiple substituents, there may be a plurality of identical substituents, or one or a group of a plurality of different substituents.

### DETAILED DESCRIPTION

The present disclosure is further described below with reference to examples, but these examples are not intended to limit the scope of the present disclosure. In the examples of the present disclosure, experimental methods without specific conditions specified were generally conducted under conventional conditions, such as those outlined in Antibodies: A Laboratory Manual and Molecular Cloning: A Laboratory Manual by Cold Spring Harbor Laboratory, or under conditions recommended by the manufacturers of the starting materials or commercial products. Reagents without specific sources indicated were commercially available conventional reagents.

### Example 1. Design of Human APP RNAi Agents

With the human APP gene (NM_000484.3) as a target gene, RNAi agents of 19/21nt were designed in accordance with the general rules for active RNAi agents. The sequences of unmodified sense and antisense strands are shown in Tables 2a and 2b, and the sequences of modified sense and antisense strands are detailed in Table 3.

**Table 2a. The sense and antisense strands of RNAi agents of the present disclosure**

| Double strand No. | SEQ ID NO: | SS strand (5'-3') | SEQ ID NO: | AS strand (5'-3') |
|---|---|---|---|---|
| TJR103586 | 1 | AGUCUACCCUGAACUGCAG | 12 | CUGCAGUUCAGGGUAGACUUC |
| TJR103579 | 2 | AGUCUACCCUGAACUGCAA | 13 | UUGCAGUUCAGGGUAGACUUC |
| TJR103778 | 3 | UGACGAGGACGAUGAGGAU | 14 | AUCCUCAUCGUCCUCGUCAUC |
| TJR103580 | 4 | UGACGAGGACGAUGAGGAA | 15 | UUCCUCAUCGUCCUCGUCAUC |
| TJR103581 | 5 | GAACGUGGGAGUUCAGCUA | 16 | UAGCUGAACUCCCACGUUCAC |
| TJR103779 | 6 | UUAAGUAUUUCAGAUGCUU | 17 | AAGCAUCUGAAAUACUUAAAA |
| TJR103582 | 7 | UUAAGUAUUUCAGAUGCUA | 18 | UAGCAUCUGAAAUACUUAAAA |
| TJR103780 | 8 | GGGGUGCUCUGCUGGUCUU | 19 | AAGACCAGCAGAGCACCCCUC |
| TJR103583 | 9 | GGGGUGCUCUGCUGGUCUA | 20 | UAGACCAGCAGAGCACCCCUC |
| TJR103584 | 10 | GGUGCUCUGCUGGUCUUCA | 21 | UGAAGACCAGCAGAGCACCCC |
| TJR103585 | 11 | GUGCUCUGCUGGUCUUCAA | 22 | UUGAAGACCAGCAGAGCACCC |
| TJR103587 | 229 | GUCUACCCUGAACUGCAGA | 234 | UCUGCAGUUCAGGGUAGACUU |
| TJR103589 | 230 | GAUGACGAGGACGAUGAGG | 235 | CCUCAUCGUCCUCGUCAUCAU |
| TJR103592 | 231 | GUGAACGUGGGAGUUCAGC | 236 | GCUGAACUCCCACGUUCACAU |
| TJR103593 | 232 | UUUUAAGUAUUUCAGAUGC | 237 | GCAUCUGAAAUACUUAAAAAU |
| TJR103596 | 233 | AGGGGUGCUCUGCUGGUCU | 238 | AGACCAGCAGAGCACCCCUCC |

**Table 2b. The sense and antisense strands of RNAi agents of the present disclosure**

| SEQ ID NO: | Unmodified SS strand (5'-3') | SEQ ID NO: | Unmodified AS strand (5'-3') |
|---|---|---|---|
| 23 | CGAGGACGAUGAGGAUGGU | 66 | ACCAUCCUCAUCGUCCUCGUC |
| 24 | GGACGAUGAGGAUGGUGAU | 67 | AUCACCAUCCUCAUCGUCCUC |
| 25 | CGAUGAGGAUGGUGAUGAA | 68 | UUCAUCACCAUCCUCAUCGUC |
| 26 | GGCUGAGGAACCCUACGAA | 69 | UUCGUAGGGUUCCUCAGCCUC |
| 27 | GCUGAGGAACCCUACGAAA | 70 | UUUCGUAGGGUUCCUCAGCCU |
| 28 | CUGAGGAACCCUACGAAGA | 71 | UCUUCGUAGGGUUCCUCAGCC |
| 29 | UGAGGAACCCUACGAAGAA | 72 | UUCUUCGUAGGGUUCCUCAGC |
| 30 | GAGGAACCCUACGAAGAAA | 73 | UUUCUUCGUAGGGUUCCUCAG |
| 31 | GAACCCUACGAAGAAGCCA | 74 | UGGCUUCUUCGUAGGGUUCCU |
| 32 | ACCCUACGAAGAAGCCACA | 75 | UGUGGCUUCUUCGUAGGGUUC |
| 33 | CCUACGAAGAAGCCACAGA | 76 | UCUGUGGCUUCUUCGUAGGGU |
| 34 | UACGAAGAAGCCACAGAGA | 77 | UCUCUGUGGCUUCUUCGUAGG |
| 35 | CGAAGAAGCCACAGAGAGA | 78 | UCUCUCUGUGGCUUCUUCGUA |
| 36 | GAAGAAGCCACAGAGAGAA | 79 | UUCUCUCUGUGGCUUCUUCGU |
| 37 | GAAGCCACAGAGAGAACCA | 80 | UGGUUCUCUCUGUGGCUUCUU |
| 38 | GCCACAGAGAGAACCACCA | 81 | UGGUGGUUCUCUCUGUGGCUU |
| 39 | CCACAGAGAGAACCACCAA | 82 | UUGGUGGUUCUCUCUGUGGCU |
| 40 | GCCACCACCACCACCACCA | 83 | UGGUGGUGGUGGUGGUGGCAA |
| 41 | GAGAGGUGUGCUCUGAACA | 84 | UGUUCAGAGCACACCUCUCGA |
| 42 | AGAGGUGUGCUCUGAACAA | 85 | UUGUUCAGAGCACACCUCUCG |
| 43 | GGCCGUGCCGAGCAAUGAU | 86 | AUCAUUGCUCGGCACGGCCCC |
| 44 | CCGUGCCGAGCAAUGAUCU | 87 | AGAUCAUUGCUCGGCACGGCC |
| 45 | CGUGCCGAGCAAUGAUCUA | 88 | UAGAUCAUUGCUCGGCACGGC |
| 46 | GGAUGUGGCGGCAACCGGA | 89 | UCCGGUUGCCGCCACAUCCGC |
| 47 | GAUGUGGCGGCAACCGGAA | 90 | UUCCGGUUGCCGCCACAUCCG |
| 48 | GGCGGCAACCGGAACAACU | 91 | AGUUGUUCCGGUUGCCGCCAC |
| 49 | GCGGCAACCGGAACAACUU | 92 | AAGUUGUUCCGGUUGCCGCCA |
| 50 | CGGCAACCGGAACAACUUU | 93 | AAAGUUGUUCCGGUUGCCGCC |
| 51 | GGCAACCGGAACAACUUUA | 94 | UAAAGUUGUUCCGGUUGCCGC |
| 52 | GCAACCGGAACAACUUUGA | 95 | UCAAAGUUGUUCCGGUUGCCG |
| 53 | AGAACGUCAAGCAAAGAAA | 96 | UUUCUUUGCUUGACGUUCUGC |
| 54 | GGCCAGAGUGGAAGCCAUA | 97 | UAUGGCUUCCACUCUGGCCAU |
| 55 | AGAACAGAAGGACAGACAA | 98 | UUGUCUGUCCUUCUGUUCUGC |
| 56 | UCACAUAGCCCCUUAGCCA | 99 | UGGCUAAGGGGCUAUGUGAUA |
| 57 | GCCCCUUAGCCAGUUGUAU | 100 | AUACAACUGGCUAAGGGGCUA |
| 58 | CCCCUUAGCCAGUUGUAUA | 101 | UAUACAACUGGCUAAGGGGCU |
| 59 | AUGUGAACGUGGGAGUUCA | 102 | UGAACUCCCACGUUCACAUGA |
| 60 | UAAGUAUUUCAGAUGCUUU | 103 | AAAGCAUCUGAAAUACUUAAA |
| 61 | GGAAUUAAGAGGAUACACA | 104 | UGUGUAUCCUCUUAAUUCCUA |
| 62 | GAAUUAAGAGGAUACACAA | 105 | UUGUGUAUCCUCUUAAUUCCU |
| 63 | GCAUUGAGACUUCAAGCUU | 106 | AAGCUUGAAGUCUCAAUGCCU |
| 64 | CAUUGAGACUUCAAGCUUU | 107 | AAAGCUUGAAGUCUCAAUGCC |
| 65 | GGAGGGGUGCUCUGCUGGU | 108 | ACCAGCAGAGCACCCCUCCCC |

**Table 3. The sense and antisense strands of RNAi agents of the present disclosure**

| Double strand No. | SEQ ID NO: | SS strand (5'-3') | SEQ ID NO: | AS strand (5'-3') |
|---|---|---|---|---|
| TJR101344 | 109 | AmsGmsUmCmUmAmCfCfCfUm GmAmAmCmUmGmCmAmAm | 126 | VPUmsUfsGmCmAmGfUmUmC mAmGmGfGmUfAmGfAmCmU msUmsCm |
| TJR101345 | 110 | UmsGmsAmCmGmAmGfGfAfCm GmAmUmGmAmGmGmAmAm | 127 | VPUmsUfsCmCmUmCfAmUmC mGmUmCfCmUfCmGfUmCmAm sUmsCm |
| TJR101383 | 111 | GmsAmsAmCmGmUmGfGfGfAm GmUmUmCmAmGmCmUmAm | 128 | VPUmsAfsGmCmUmGfAmAmC mUmCmCfCmAfCmGfUmUmCm sAmsCm |
| TJR101384 | 112 | UmsUmsAmAmGmUmAfUfUfUm CmAmGmAmUmGmCmUmAm | 129 | VPUmsAfsGmCmAmUfCmUmG mAmAmAfUmAfCmUfUmAmA msAmsAm |
| TJR101391 | 113 | GmsGmsGmGmUmGmCfUfCfUm GmCmUmGmGmUmCmUmAm | 130 | VPUmsAfsGmAmCmCfAmGmC mAmGmAfGmCfAmCfCmCmCm sUmsCm |
| TJR101392 | 114 | GmsGmsUmGmCmUmCfUfGfCm UmGmGmUmCmUmUmCmAm | 131 | VPUmsGfsAmAmGmAfCmCmA mGmCmAfGmAfGmCfAmCmCm sCmsCm |
| TJR101393 | 115 | GmsUmsGmCmUmCmUfGfCfUm GmGmUmCmUmUmCmAmAm | 132 | VPUmsUfsGmAmAmGfAmCmC mAmGmCfAmGfAmGfCmAmCm sCmsCm |
| TJR103639 | 116 | AhdsGmsUmCmUmAmCfCfCfUm GmAmAmCmUmGmCmsAmsAm | 133 | UmsUfsGmCfAmGfUmUmCmAf GmGfGmUfAmGfAmCfUmsUms Cm |
| TJR103640 | 117 | UhdsGmsAmCmGmAmGfGfAfC mGmAmUmGmAmGmGmsAmsA m | 134 | UmsUfsCmCfUmCfAmUmCmGf UmCfCmUfCmGfUmCfAmsUms Cm |
| TJR103641 | 118 | GhdsAmsAmCmGmUmGfGfGfA mGmUmUmCmAmGmCmsUmsA m | 135 | UmsAfsGmCfUmGfAmAmCmUf CmCfCmAfCmGfUmUfCmsAms Cm |
| TJR103642 | 119 | UhdsUmsAmAmGmUmAfUfUfU mCmAmGmAmUmGmCmsUmsA m | 136 | UmsAfsGmCfAmUfCmUmGmAf AmAfUmAfCmUfUmAfAmsAms Am |
| TJR103643 | 120 | GhdsGmsGmGmUmGmCfUfCfU mGmCmUmGmGmUmCmsUmsA m | 137 | UmsAfsGmAfCmCfAmGmCmAf GmAfGmCfAmCfCmCfCmsUms Cm |
| TJR103644 | 121 | GhdsGmsUmGmCmUmCfUfGfCm UmGmGmUmCmUmUmsCmsAm | 138 | UmsGfsAmAfGmAfCmCmAmGf CmAfGmAfGmCfAmCfCmsCms Cm |
| TJR103645 | 122 | GhdsUmsGmCmUmCmUfGfCfUm GmGmUmCmUmUmCmsAmsAm | 139 | UmsUfsGmAfAmGfAmCmCmAf GmCfAmGfAmGfCmAfCmsCms Cm |
| TJR103797 | 123 | UmsUmsAmAmGmNA0133’UAf UfUfUmCmAmGmAmUmGmCms UmsAm | 140 | VPUmsAfsGmCmAmUfCmUmG mAmAmAfUmAfCmUfUmAmA msAmsAm |
| TJR103798 | 124 | NA0133’UsUmsAmAmGmUmAf UfUfUmCmAmGmAmUmGmCms UmsAm | 141 | VPUmsAfsGmCmAmUfCmUmG mAmAmAfUmAfCmUfUmAmA msAmsAm |
| TJR103799 | 125 | UhdsUmsAmAmGmUmAfUfUfU mCmAmGmAmUmGmCmsUmsA m | 142 | VPUmsAfsGmCmAmUfCmUmG mAmAmAfUmAfCmUfUmAmA msAmsAm |
| TJR101346 | 143 | CmsGmsAmGmGmAmCfGfAfUm GmAmGmGmAmUmGmGmAm | 186 | VPUmsCfsCmAmUmCfCmUmC mAmUmCfGmUfCmCfUmCmGm sUmsCm |
| TJR101347 | 144 | GmsGmsAmCmGmAmUfGfAfGm GmAmUmGmGmUmGmAmAm | 187 | VPUmsUfsCmAmCmCfAmUmC mCmUmCfAmUfCmGfUmCmCm sUmsCm |
| TJR101348 | 145 | CmsGmsAmUmGmAmGfGfAfUm GmGmUmGmAmUmGmAmAm | 188 | VPUmsUfsCmAmUmCfAmCmC mAmUmCfCmUfCmAfUmCmGm sUmsCm |
| TJR101349 | 146 | GmsGmsCmUmGmAmGfGfAfAm CmCmCmUmAmCmGmAmAm | 189 | VPUmsUfsCmGmUmAfGmGmG mUmUmCfCmUfCmAfGmCmCm sUmsCm |
| TJR101350 | 147 | GmsCmsUmGmAmGmGfAfAfCm CmCmUmAmCmGmAmAmAm | 190 | VPUmsUfsUmCmGmUfAmGmG mGmUmUfCmCfUmCfAmGmCm sCmsUm |
| TJR101351 | 148 | CmsUmsGmAmGmGmAfAfCfCm CmUmAmCmGmAmAmGmAm | 191 | VPUmsCfsUmUmCmGfUmAmG mGmGmUfUmCfCmUfCmAmGm sCmsCm |
| TJR101352 | 149 | UmsGmsAmGmGmAmAfCfCfCm UmAmCmGmAmAmGmAmAm | 192 | VPUmsUfsCmUmUmCfGmUmA mGmGmGfUmUfCmCfUmCmAm sGmsCm |
| TJR101353 | 150 | GmsAmsGmGmAmAmCfCfCfUm AmCmGmAmAmGmAmAmAm | 193 | VPUmsUfsUmCmUmUfCmGmU mAmGmGfGmUfUmCfCmUmCm sAmsGm |
| TJR101354 | 151 | GmsAmsAmCmCmCmUfAfCfGm AmAmGmAmAmGmCmCmAm | 194 | VPUmsGfsGmCmUmUfCmUmU mCmGmUfAmGfGmGfUmUmCm sCmsUm |
| TJR101355 | 152 | AmsCmsCmCmUmAmCfGfAfAm GmAmAmGmCmCmAmCmAm | 195 | VPUmsGfsUmGmGmCfUmUmC mUmUmCfGmUfAmGfGmGmU msUmsCm |
| TJR101356 | 153 | CmsCmsUmAmCmGmAfAfGfAm AmGmCmCmAmCmAmGmAm | 196 | VPUmsCfsUmGmUmGfGmCmU mUmCmUfUmCfGmUfAmGmGm sGmsUm |
| TJR101357 | 154 | UmsAmsCmGmAmAmGfAfAfGm CmCmAmCmAmGmAmGmAm | 197 | VPUmsCfsUmCmUmGfUmGmG mCmUmUfCmUfUmCfGmUmAm sGmsGm |
| TJR101358 | 155 | CmsGmsAmAmGmAmAfGfCfCm AmCmAmGmAmGmAmGmAm | 198 | VPUmsCfsUmCmUmCfUmGmU mGmGmCfUmUfCmUfUmCmGm sUmsAm |
| TJR101359 | 156 | GmsAmsAmGmAmAmGfCfCfAm CmAmGmAmGmAmGmAmAm | 199 | VPUmsUfsCmUmCmUfCmUmG mUmGmGfCmUfUmCfUmUmCm sGmsUm |
| TJR101360 | 157 | GmsAmsAmGmCmCmAfCfAfGm AmGmAmGmAmAmCmCmAm | 200 | VPUmsGfsGmUmUmCfUmCmU mCmUmGfUmGfGmCfUmUmCm sUmsUm |
| TJR101361 | 158 | GmsCmsCmAmCmAmGfAfGfAm GmAmAmCmCmAmCmCmAm | 201 | VPUmsGfsGmUmGmGfUmUmC mUmCmUfCmUfGmUfGmGmCm sUmsUm |
| TJR101362 | 159 | CmsCmsAmCmAmGmAfGfAfGm AmAmCmCmAmCmCmAmAm | 202 | VPUmsUfsGmGmUmGfGmUmU mCmUmCfUmCfUmGfUmGmGm sCmsUm |
| TJR101363 | 160 | GmsCmsCmAmCmCmAfCfCfAm CmCmAmCmCmAmCmCmAm | 203 | VPUmsGfsGmUmGmGfUmGmG mUmGmGfUmGfGmUfGmGmC msAmsAm |
| TJR101364 | 161 | GmsAmsGmAmGmGmUfGfUfGm CmUmCmUmGmAmAmCmAm | 204 | VPUmsGfsUmUmCmAfGmAmG mCmAmCfAmCfCmUfCmUmCm sGmsAm |
| TJR101365 | 162 | AmsGmsAmGmGmUmGfUfGfCm UmCmUmGmAmAmCmAmAm | 205 | VPUmsUfsGmUmUmCfAmGmA mGmCmAfCmAfCmCfUmCmUm sCmsGm |
| TJR101366 | 163 | GmsGmsCmCmGmUmGfCfCfGm AmGmCmAmAmUmGmAmAm | 206 | VPUmsUfsCmAmUmUfGmCmU mCmGmGfCmAfCmGfGmCmCm sCmsCm |
| TJR101367 | 164 | CmsCmsGmUmGmCmCfGfAfGm CmAmAmUmGmAmUmCmAm | 207 | VPUmsGfsAmUmCmAfUmUmG mCmUmCfGmGfCmAfCmGmGm sCmsCm |
| TJR101368 | 165 | CmsGmsUmGmCmCmGfAfGfCm AmAmUmGmAmUmCmUmAm | 208 | VPUmsAfsGmAmUmCfAmUmU mGmCmUfCmGfGmCfAmCmGm sGmsCm |
| TJR101369 | 166 | GmsGmsAmUmGmUmGfGfCfGm GmCmAmAmCmCmGmGmAm | 209 | VPUmsCfsCmGmGmUfUmGmC mCmGmCfCmAfCmAfUmCmCm sGmsCm |
| TJR101370 | 167 | GmsAmsUmGmUmGmGfCfGfGm CmAmAmCmCmGmGmAmAm | 210 | VPUmsUfsCmCmGmGfUmUmG mCmCmGfCmCfAmCfAmUmCm sCmsGm |
| TJR101371 | 168 | GmsGmsCmGmGmCmAfAfCfCm GmGmAmAmCmAmAmCmAm | 211 | VPUmsGfsUmUmGmUfUmCmC mGmGmUfUmGfCmCfGmCmCm sAmsCm |
| TJR101372 | 169 | GmsCmsGmGmCmAmAfCfCfGm GmAmAmCmAmAmCmUmAm | 212 | VPUmsAfsGmUmUmGfUmUmC mCmGmGfUmUfGmCfCmGmCm sCmsAm |
| TJR101373 | 170 | CmsGmsGmCmAmAmCfCfGfGm AmAmCmAmAmCmUmUmAm | 213 | VPUmsAfsAmGmUmUfGmUmU mCmCmGfGmUfUmGfCmCmGm sCmsCm |
| TJR101374 | 171 | GmsGmsCmAmAmCmCfGfGfAm AmCmAmAmCmUmUmUmAm | 214 | VPUmsAfsAmAmGmUfUmGmU mUmCmCfGmGfUmUfGmCmCm sGmsCm |
| TJR101375 | 172 | GmsCmsAmAmCmCmGfGfAfAm CmAmAmCmUmUmUmGmAm | 215 | VPUmsCfsAmAmAmGfUmUmG mUmUmCfCmGfGmUfUmGmCm sCmsGm |
| TJR101376 | 173 | AmsGmsAmAmCmGmUfCfAfAm GmCmAmAmAmGmAmAmAm | 216 | VPUmsUfsUmCmUmUfUmGmC mUmUmGfAmCfGmUfUmCmUm sGmsCm |
| TJR101377 | 174 | GmsGmsCmCmAmGmAfGfUfGm GmAmAmGmCmCmAmUmAm | 217 | VPUmsAfsUmGmGmCfUmUmC mCmAmCfUmCfUmGfGmCmCm sAmsUm |
| TJR101378 | 175 | AmsGmsAmAmCmAmGfAfAfGm GmAmCmAmGmAmCmAmAm | 218 | VPUmsUfsGmUmCmUfGmUmC mCmUmUfCmUfGmUfUmCmUm sGmsCm |
| TJR101379 | 176 | UmsCmsAmCmAmUmAfGfCfCm CmCmUmUmAmGmCmCmAm | 219 | VPUmsGfsGmCmUmAfAmGmG mGmGmCfUmAfUmGfUmGmA msUmsAm |
| TJR101380 | 177 | GmsCmsCmCmCmUmUfAfGfCm CmAmGmUmUmGmUmAmAm | 220 | VPUmsUfsAmCmAmAfCmUmG mGmCmUfAmAfGmGfGmGmCm sUmsAm |
| TJR101381 | 178 | CmsCmsCmCmUmUmAfGfCfCm AmGmUmUmGmUmAmUmAm | 221 | VPUmsAfsUmAmCmAfAmCmU mGmGmCfUmAfAmGfGmGmG msCmsUm |
| TJR101382 | 179 | AmsUmsGmUmGmAmAfCfGfUm GmGmGmAmGmUmUmCmAm | 222 | VPUmsGfsAmAmCmUfCmCmC mAmCmGfUmUfCmAfCmAmUm sGmsAm |
| TJR101385 | 180 | UmsAmsAmGmUmAmUfUfUfCm AmGmAmUmGmCmUmUmAm | 223 | VPUmsAfsAmGmCmAfUmCmU mGmAmAfAmUfAmCfUmUmA msAmsAm |
| TJR101386 | 181 | GmsGmsAmAmUmUmAfAfGfAm GmGmAmUmAmCmAmCmAm | 224 | VPUmsGfsUmGmUmAfUmCmC mUmCmUfUmAfAmUfUmCmCm sUmsAm |
| TJR101387 | 182 | GmsAmsAmUmUmAmAfGfAfGm GmAmUmAmCmAmCmAmAm | 225 | VPUmsUfsGmUmGmUfAmUmC mCmUmCfUmUfAmAfUmUmCm sCmsUm |
| TJR101388 | 183 | GmsCmsAmUmUmGmAfGfAfCm UmUmCmAmAmGmCmUmAm | 226 | VPUmsAfsGmCmUmUfGmAmA mGmUmCfUmCfAmAfUmGmCm sCmsUm |
| TJR101389 | 184 | CmsAmsUmUmGmAmGfAfCfUm UmCmAmAmGmCmUmUmAm | 227 | VPUmsAfsAmGmCmUfUmGmA mAmGmUfCmUfCmAfAmUmGm sCmsCm |
| TJR101390 | 185 | GmsGmsAmGmGmGmGfUfGfCm UmCmUmGmCmUmGmGmAm | 228 | VPUmsCfsCmAmGmCfAmGmA mGmCmAfCmCfCmCfUmCmCm sCmsCm |

In Tables 2a, 2b, and 3 above, the sequences are shown in the direction from the 5' end to the 3' end (left to right); "G", "C", "A", "T", and "U" represent nucleosides, and comprise the bases of guanine, cytosine, adenine, thymidine, and uracil, respectively. The lowercase letter m means that the nucleoside adjacent to the letter m on the left side is a 2'-methoxy-modified nucleoside; the lowercase letter f means that the nucleoside adjacent to the letter f on the left side is a 2'-fluoro-modified nucleoside; VP means that the 5' end of the nucleoside adjacent to the letters on the left/right side is a trans-vinylphosphodiester group (5'-*E*-VP); the lowercase letters hd mean that the nucleoside adjacent to the letters hd on the left side is a nucleoside with -O-n-hexadecyl linked to the 2' position of the sugar ring; NA0133' means that the nucleoside adjacent to NA0133' on the right side is a nucleoside with -CH₂O-n-hexadecyl linked to the 2' position of the sugar ring, i.e., having the structure represented by formula (3) in the specification; the lowercase letter s means that the two nucleosides adjacent to the letter s are linked by a phosphorothioate diester group; unless otherwise specified, two adjacent nucleosides are linked by a phosphodiester group. Unless otherwise specified, the 3' position of the first nucleotide at the 3' end of each strand is hydroxy; the 5' position of the first nucleotide at the 5' end of each strand is hydroxy.

The structures of the 2'-methoxy-modified nucleoside, the 2'-fluoro-modified nucleoside, the nucleoside with -O-n-hexadecyl linked to the 2'-position of the sugar ring, the trans-vinylphosphodiester group (5'-*E*-VP), the phosphorothioate diester group, the nucleoside with -CH₂O-n-hexadecyl linked to the 2' position of the sugar ring, and the phosphodiester group are shown in the table below. When the RNAi agents of the present disclosure are present in the form of a salt, for example, in the form of a sodium salt, the structures of the salt forms corresponding to the structures in Table 4 below also fall within the protection scope of the present disclosure. In Table 4, Base represents a base at the corresponding position:

**Table 4**

| Structure | Name |
|---|---|
| 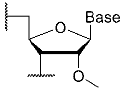 | 2'-Methoxy-modified nucleoside |
| 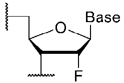 | 2'-Fluoro-modified nucleoside |
| 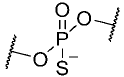 | Anion form of phosphorothioate diester group |
| 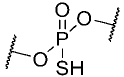 | Phosphorothioate diester group |
| 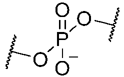 | Anion form of phosphodiester group |
| 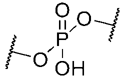 | Phosphodiester group |
| 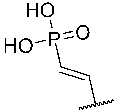 | *E*-Vinylphosphoric acid group |
| | (E-VP) |
| 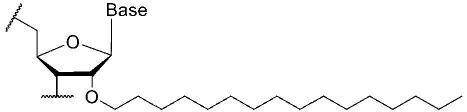 | Nucleoside with -O-n-hexadecyl linked to 2' position of sugar ring |
| 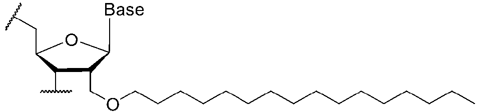 | Nucleoside with -CH₂O-n-hexadecyl linked to 2' position of sugar ring |

### Example 2. Synthesis of RNAi Agents

The synthesis of RNAi agents of the present disclosure does not differ from the conventional phosphoramidite solid-phase synthesis method. The synthesis process is briefly described below: Nucleoside phosphoramidite monomers were linked one by one according to a synthesis program on a Dr. Oligo48 synthesizer (Biolytic), with a universal CPG support as a start. The nucleoside monomer starting materials, including nucleoside phosphoramidite monomers such as nucleoside phosphoramidite monomers with *E*-VP at the 5' position, 2'-methoxy-modified nucleoside phosphoramidite monomers, 2'-fluoro-modified nucleoside phosphoramidite monomers, E-vinylphosphoric acid group-containing nucleoside phosphoramidite monomers, and nucleoside phosphoramidite monomers with -O-n-hexadecyl linked to the 2' position of the sugar ring, were purchased from Shanghai Hongene, Suzhou Genepharma, or Jiangsu Synthgene. 5-Ethylthio-1H-tetrazole (ETT) was used as an activator (0.6 M in acetonitrile), a 0.22 M solution of PADS in acetonitrile and collidine (1:1 by volume) (Suzhou Kroma) was used as a sulfurizing agent, and an iodopyridine/water solution (Kroma) was used as an oxidant.

After solid-phase synthesis was complete, the oligoribonucleotides were cleaved from the solid support by soaking in a solution of 28% ammonia water and ethanol (3:1) at 50 °C for 16 h. Centrifugation was then performed, and the supernatant was transferred to another centrifuge tube. After the supernatant was concentrated to dryness by evaporation, the residue was purified by C18 reversed-phase chromatography with 0.1 M TEAA and acetonitrile as the mobile phase, and DMTr was removed using a 3% trifluoroacetic acid solution. The target oligonucleotides were collected, then lyophilized, identified as the target products by LC-MS, and quantified by UV (260 nm).

The single-stranded oligonucleotides obtained were complementarily paired in an equimolar ratio and annealed. The final RNAi agents were dissolved in 1× PBS, and the concentration of the solutions was adjusted to the concentration required for the experiment so that they were ready for use.

The RNAi agents containing NA0133' can be prepared using the method described in PCT Application No. WO2024125556A1.

### Example 3. Inhibition of Human APP in MCF-7 Cells by RNAi Agents - Inhibitory Activity at 3 Concentration Points

The RNAi agents were subjected to *in vitro* molecular-level activity screening in MCF-7 cells using 3 concentration gradients (10 nM, 1 nM, and 0.1 nM).

MCF-7 cells were cultured at 37 °C with 5% CO₂ in a Dulbecco's modified eagle medium containing 10% fetal bovine serum. 24 h prior to transfection, the MCF-7 cells were seeded into a 96-well plate at a density of 1 × 10⁴ cells per well, with each well containing 100 µL of the culture medium.

The cells were transfected with the RNAi agents using Lipofectamine RNAi MAX (ThermoFisher, 13778150) according to the product instructions. The final gradient concentrations of the RNAi agents for transfection were 10 nM, 1 nM, and 0.1 nM, and two duplicate wells were set for each concentration. 24 h after treatment, total cell RNA extraction was performed using an FG0417-L/FG0418-XL high-throughput cell RNA extraction kit (FireGen, magnetic bead method), and RNA reverse transcription (Takara, RR037A) and quantitative real-time PCR (Thermo, 4444557) detection were performed to determine the mRNA level of human APP. The mRNA level of human APP was corrected based on the GAPDH internal reference gene level.

The instruments involved in the experiment are shown in Table 5.

**Table 5. Experimental instruments**

| Name | Company | Cat. No./model |
|---|---|---|
| Nanodrop | Thermo | Nanodrop One |
| qPCR system | ABI | 7500 Fast |
| PCR | Life | Pro-Flex PCR system |

For the real-time quantitative PCR detection, a probe Q-PCR detection experiment was used. Information on the primers is shown in Table 6.

**Table 6. Taqman primers**

| Primer name | SEQ ID NO | Primer sequence (5'-3') |
|---|---|---|
| hAPP-Forward | 239 | GACAGACAGCACACCCTAAA |
| hAPP-Reverse | 240 | CACACGGAGGTGTGTCATAA |
| hAPP-Probe | 241 | 5`6-FAM-ATCCCAAGAAAGCCGCTCAGATCC- 3`BHQ1 |
| hGAPDH-Forward | 242 | GACCCCTTCATTGACCTCAACTAC |
| hGAPDH-Reverse | 243 | TTGACGGTGCCATGGAATTT |
| hGAPDH-Probe | 244 | 5`VIC-TTACATGTTCCAATATGATTCC-3`BHQ1 |

### Result analysis method

After the Q-PCR detection experiment was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as 2^{-△△Ct}, where △△Ct = [(target gene of Ct experimental group - internal reference of Ct experimental group) - (target gene of Ct control group - internal reference of Ct control group)]. Inhibition rate (%) = (1 - remaining level of target gene expression) × 100%. The results are shown in Table 7. The results of this example are expressed relative to the remaining percentage of human APP mRNA expression in cells treated with the control RNAi agent.

**Table 7. The single-dose inhibitory activity of RNAi agents in MCF-7 cells**

| Double strand No. | 10 nM Remaining percentage of expression | STDEV | 1 nM Remaining percentage of expression | STDEV | 0.1 nM Remaining percentage of expression | STDEV |
|---|---|---|---|---|---|---|
| TJR101344 | 2.88% | 0.29% | 3.44% | 0.17% | 8.98% | 0.68% |
| TJR101345 | 2.80% | 0.14% | 4.00% | 0.18% | 17.77% | 0.69% |
| TJR101346 | 3.87% | 0.06% | 7.64% | 0.13% | 34.40% | 0.81% |
| TJR101347 | 3.44% | 0.11% | 4.63% | 0.38% | 18.06% | 0.12% |
| TJR101352 | 3.37% | 0.05% | 4.84% | 0.26% | 22.54% | 1.06% |
| TJR101353 | 6.22% | 0.10% | 10.40% | 0.68% | 40.31% | 1.37% |
| TJR101354 | 17.54% | 0.53% | 31.40% | 1.38% | 75.04% | 0.16% |
| TJR101355 | 11.57% | 0.83% | 25.69% | 0.08% | 76.04% | 0.23% |
| TJR101348 | 4.08% | 1.17% | 4.46% | 0.67% | 14.81% | 2.55% |
| TJR101349 | 7.89% | 0.14% | 9.52% | 0.17% | 28.90% | 0.56% |
| TJR101350 | 6.65% | 0.36% | 11.97% | 0.08% | 44.45% | 0.97% |
| TJR101351 | 7.58% | 0.01% | 13.51% | 0.12% | 51.20% | 1.59% |
| TJR101356 | 22.40% | 0.06% | 48.02% | 4.04% | 84.01% | 0.78% |
| TJR101357 | 16.98% | 0.85% | 26.68% | 0.65% | 62.72% | 0.88% |
| TJR101358 | 4.34% | 0.02% | 6.74% | 0.45% | 24.88% | 1.04% |
| TJR101359 | 5.64% | 0.59% | 8.22% | 0.25% | 33.95% | 2.85% |
| TJR101360 | 10.89% | 0.65% | 18.15% | 2.82% | 53.89% | 2.13% |
| TJR101361 | 11.00% | 0.47% | 16.79% | 0.12% | 48.71% | 4.64% |
| TJR101362 | 10.43% | 0.10% | 20.38% | 0.37% | 62.27% | 2.69% |
| TJR101363 | 86.58% | 0.30% | 93.02% | 0.74% | 102.65% | 4.84% |
| TJR101371 | 27.74% | 0.75% | 39.62% | 1.98% | 79.49% | 0.23% |
| TJR101372 | 18.67% | 1.07% | 22.99% | 0.26% | 49.38% | 1.91% |
| TJR101373 | 20.96% | 0.77% | 28.46% | 0.44% | 64.97% | 3.03% |
| TJR101374 | 29.71% | 0.18% | 32.29% | 1.94% | 71.06% | 4.97% |
| TJR101365 | 15.72% | 0.25% | 16.09% | 0.20% | 34.00% | 2.60% |
| TJR101366 | 33.84% | 0.20% | 53.49% | 3.08% | 90.63% | 1.90% |
| TJR101368 | 17.02% | 0.33% | 19.45% | 0.03% | 47.47% | 0.92% |
| TJR101370 | 41.23% | 0.42% | 51.46% | 0.70% | 89.02% | 1.75% |
| TJR101375 | 19.76% | 0.10% | 24.46% | 0.09% | 60.26% | 1.44% |
| TJR101376 | 4.11% | 0.35% | 5.76% | 0.07% | 24.86% | 0.40% |
| TJR101377 | 16.09% | 0.88% | 30.63% | 1.10% | 90.93% | 0.29% |
| TJR101378 | 9.57% | 0.38% | 12.11% | 0.24% | 32.00% | 1.11% |
| TJR101379 | 4.30% | 0.33% | 6.46% | 0.20% | 19.97% | 0.76% |
| TJR101380 | 6.13% | 0.39% | 6.35% | 0.34% | 14.26% | 0.53% |
| TJR101381 | 6.72% | 0.34% | 7.15% | 0.09% | 13.32% | 0.12% |
| TJR101382 | 3.44% | 0.10% | 5.94% | 0.01% | 21.04% | 0.20% |
| TJR101387 | 6.57% | 0.18% | 6.12% | 0.20% | 10.43% | 0.15% |
| TJR101388 | 4.91% | 0.11% | 5.76% | 0.29% | 10.87% | 0.16% |
| TJR101389 | 4.99% | 0.03% | 6.14% | 0.16% | 10.86% | 0.22% |
| TJR101390 | 10.59% | 0.19% | 19.28% | 0.28% | 53.61% | 0.07% |
| TJR101383 | 5.01% | 0.83% | 6.31% | 0.24% | 14.83% | 0.70% |
| TJR101384 | 4.57% | 0.18% | 5.15% | 0.07% | 7.24% | 0.02% |
| TJR101385 | 3.88% | 0.19% | 6.99% | 0.47% | 21.65% | 0.78% |
| TJR101386 | 5.31% | 0.23% | 5.75% | 0.25% | 10.88% | 0.04% |
| TJR101391 | 8.30% | 0.15% | 9.81% | 0.38% | 20.45% | 0.65% |
| TJR101392 | 6.97% | 0.26% | 8.44% | 0.47% | 14.06% | 0.48% |
| TJR101393 | 6.36% | 0.28% | 6.30% | 0.20% | 10.86% | 0.87% |

### Example 4. Inhibition of Human APP in MCF-7 Cells by RNAi Agents

According to the method in Example 3, the RNAi agent sequences were subjected to *in vitro* molecular-level activity screening in MCF-7 cells using 9 concentration gradients. The results of this example are shown in Table 8. The results are expressed relative to the remaining percentage of human APP mRNA expression in cells treated with the control RNAi agent. The inhibition rate IC₅₀ results are shown in Table 8.

**Table 8. The activity of RNAi agents at 9 concentration points in MCF-7 cells (IC₅₀)**

| Remaining percentage of APP mRNA expression (mean) | | | | | |
|---|---|---|---|---|---|
| Double strand code | 10.0000 nM | 1.6667 nM | 0.2778 nM | 0.0463 nM | 0.0077 nM |
| TJR101344 | 4.53 | 5.44 | 8.84 | 21.04 | 36.19 |
| TJR101345 | 6.08 | 8.85 | 19.70 | 43.27 | 69.14 |
| TJR101347 | 8.56 | 10.35 | 18.50 | 44.99 | 77.38 |
| TJR101348 | 7.92 | 16.41 | 28.40 | 37.57 | 50.38 |
| TJR101379 | 8.93 | 13.12 | 22.34 | 43.83 | 69.71 |
| TJR101380 | 14.70 | 23.92 | 29.92 | 37.89 | 50.09 |
| TJR101381 | 12.31 | 18.60 | 28.34 | 37.00 | 50.91 |
| TJR101383 | 8.36 | 13.83 | 23.67 | 36.48 | 57.71 |
| TJR101384 | 5.87 | 5.85 | 9.43 | 20.49 | 34.82 |
| TJR101386 | 7.93 | 8.74 | 11.01 | 21.90 | 48.68 |
| TJR101387 | 6.91 | 7.61 | 9.43 | 19.13 | 42.75 |
| TJR101388 | 7.38 | 7.87 | 10.27 | 18.45 | 49.64 |
| TJR101389 | 8.31 | 10.11 | 14.69 | 25.31 | 46.18 |
| TJR101391 | 14.00 | 18.71 | 29.63 | 42.63 | 72.80 |
| TJR101392 | 17.25 | 27.76 | 32.46 | 39.47 | 46.63 |
| TJR101393 | 7.80 | 8.78 | 12.46 | 21.25 | 44.54 |

| Double strand code | 0.0013 nM | 0.0002 nM | 0.000036 | 0.000006 | IC₅₀ (nM) |
|---|---|---|---|---|---|
| TJR101344 | 59.46 | 76.25 | 88.81 | 91.73 | 0.0027 |
| TJR101345 | 89.34 | 98.25 | 99.13 | 100.84 | 0.0284 |
| TJR101347 | 90.30 | 96.55 | 96.53 | 96.71 | 0.0354 |
| TJR101348 | 69.62 | 89.55 | 89.27 | 92.50 | 0.0125 |
| TJR101379 | 87.80 | 98.19 | 99.58 | 97.09 | 0.0300 |
| TJR101380 | 76.34 | 95.00 | 99.05 | 101.01 | 0.0114 |
| TJR101381 | 71.65 | 94.93 | 96.69 | 101.23 | 0.0112 |
| TJR101383 | 76.13 | 86.92 | 92.76 | 97.38 | 0.0151 |
| TJR101384 | 66.59 | 81.51 | 88.79 | 95.08 | 0.0033 |
| TJR101386 | 75.84 | 93.60 | 98.79 | 100.05 | 0.0067 |
| TJR101387 | 70.81 | 88.37 | 91.14 | 97.37 | 0.0049 |
| TJR101388 | 85.84 | 100.01 | 101.24 | 100.77 | 0.0076 |
| TJR101389 | 77.54 | 92.24 | 95.11 | 99.40 | 0.0069 |
| TJR101391 | 93.43 | 93.32 | 103.82 | 98.90 | 0.0340 |
| TJR101392 | 70.11 | 92.16 | 94.45 | 96.61 | 0.0089 |
| TJR101393 | 69.14 | 85.10 | 95.16 | 96.96 | 0.0049 |

### Example 5. Inhibition of Human APP in A172 Cells by RNAi Agents - Inhibitory Activity at 7 Concentration Points

The RNAi agents of the present disclosure were subjected to *in vitro* molecular-level activity screening in A172 cells using 7 concentration gradients. The initial final concentration for transfection of each RNAi agent sample was 10 nM, and a 7-fold serial dilution was performed to obtain 7 concentration points.

A172 cells were cultured at 37 °C with 5% CO₂ in a Dulbecco's modified eagle medium containing 10% fetal bovine serum. 24 h prior to transfection, the A172 cells were seeded into a 96-well plate at a density of 1 × 10⁴ cells per well, with each well containing 100 µL of the culture medium.

The cells were transfected with the RNAi agents using Lipofectamine RNAi MAX (ThermoFisher, 13778150) according to the product instructions. A total of 7 concentration points were set for the RNAi agents, the initial concentration was 10 nM, and two duplicate wells were set for each concentration. 24 h after treatment, total cell RNA extraction was performed using an FG0417-L/FG0418-XL high-throughput cell RNA extraction kit (FireGen, magnetic bead method), and RNA reverse transcription (Takara, RR037A) and quantitative real-time PCR (Thermo, 4444557) detection were performed to determine the mRNA level of human APP. The mRNA level of human APP was corrected based on the GAPDH internal reference gene level.

The instruments involved in the experiment are shown in Table 5. For the real-time quantitative PCR detection, a probe Q-PCR detection experiment was used. Information on the primers is shown in Table 6.

### Result analysis method

After the Q-PCR detection experiment was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as 2^{-△△Ct}, where △△Ct = [(target gene of Ct experimental group - internal reference of Ct experimental group) - (target gene of Ct control group - internal reference of Ct control group)]. Inhibition rate (%) = (1 - remaining level of target gene expression) × 100%. The results are expressed relative to the remaining percentage of human APP mRNA expression in cells treated with the control RNAi agent. The inhibition rate IC₅₀ results are shown in Table 9.

**Table 9. The activity of RNAi agents at 7 concentration points in A172 cells (IC₅₀)**

| Remaining percentage of APP mRNA expression (mean) | | | | |
|---|---|---|---|---|
| Double strand code | 10.0000 nM | 1.4286 nM | 0.2041 nM | 0.0292 nM |
| TJR101344 | 2.2% | 2.2% | 3.7% | 9.4% |
| TJR101345 | 2.6% | 3.0% | 8.7% | 34.1% |
| TJR101383 | 4.4% | 4.0% | 5.8% | 16.8% |
| TJR101384 | 3.0% | 3.1% | 4.0% | 10.0% |
| TJR101391 | 6.8% | 6.2% | 7.5% | 16.9% |
| TJR101392 | 5.7% | 5.2% | 6.6% | 11.2% |
| TJR101393 | 5.0% | 4.4% | 5.4% | 9.7% |

| Double strand code | 0.0042 nM | 0.0006 nM | 0 nM | IC₅₀ (nM) |
|---|---|---|---|---|
| TJR101344 | 38.9% | 89.9% | 100.5% | 0.0027 |
| TJR101345 | 72.3% | 100.1% | 101.3% | 0.0123 |
| TJR101383 | 60.6% | 91.8% | 92.5% | 0.0062 |
| TJR101384 | 48.6% | 88.1% | 99.1% | 0.0040 |
| TJR101391 | 53.1% | 90.7% | 99.4% | 0.0047 |
| TJR101392 | 30.6% | 84.7% | 95.3% | 0.0017 |
| TJR101393 | 33.5% | 92.1% | 113.4% | 0.0022 |

### Example 6. Inhibitory Activity of RNAi Agents Against Human APP in Primary Human Hepatocytes (PHHs)

The RNAi agents in Table 10 were subjected to primary human hepatocyte (PHH) activity screening in primary human hepatocytes (PHHs) using 7 concentration gradients. The initial final concentration for transfection of each RNAi agent sample was 10 nM, and a 5-fold serial dilution was performed to obtain 7 concentration points.

PHHs were cryopreserved in liquid nitrogen. 24 h prior to transfection, the primary human hepatocytes (PHHs) were thawed and then seeded into a 96-well plate at a density of 3 × 10⁴ cells per well, with each well containing 80 µL of the culture medium.

The cells were transfected with the RNAi agents using Lipofectamine RNAi MAX (ThermoFisher, 13778150) according to the product instructions. The final gradient concentrations of the RNAi agents for transfection were 10 nM, 2 nM, 0.4 nM, 0.08 nM, 0.016 nM, 0.0032 nM, and 0.00064 nM. 24 h after treatment, total cell RNA extraction was performed using a high-throughput cell RNA extraction kit, and RNA reverse transcription and quantitative real-time PCR detection were performed to determine the mRNA level of human APP. The mRNA level of human APP was corrected based on the GAPDH internal reference gene level.

For the real-time quantitative PCR detection, a probe Q-PCR detection experiment was used. Information on the primers is shown in Table 10. The results of this example are shown in Table 10. The results in Table 10 show that the RNAi agents of the present disclosure exhibited high levels of on-target inhibitory activity against the APP gene in PHHs.

**Table 10. The activity of RNAi agents at 7 concentration points in PHHs (IC₅₀)**

| Remaining percentage of APP mRNA expression (mean) | | | | |
|---|---|---|---|---|
| Double strand code | 10.00000 nM | 2.00000 nM | 0.40000 nM | 0.08000 nM |
| TJR101344 | 8.2% | 7.3% | 7.7% | 8.4% |
| TJR101345 | 11.0% | 10.0% | 9.4% | 11.6% |
| TJR101383 | 16.9% | 15.1% | 15.9% | 24.6% |
| TJR101384 | 20.2% | 15.0% | 15.6% | 20.5% |
| TJR101391 | 25.6% | 21.8% | 23.6% | 39.9% |
| TJR101392 | 18.4% | 15.6% | 14.8% | 17.6% |
| TJR101393 | 14.3% | 11.2% | 13.8% | 17.7% |

| Double strand code | 0.01600 nM | 0.00320 nM | 0.00064 nM | IC₅₀ (nM) |
|---|---|---|---|---|
| TJR101344 | 22.8% | 45.2% | 51.5% | 0.0014 |
| TJR101345 | 31.9% | 77.8% | 69.1% | 0.0142 |
| TJR101383 | 55.3% | 101.8% | 119.7% | 0.0193 |
| TJR101384 | 64.0% | 84.5% | 105.9% | 0.0683 |
| TJR101391 | 68.0% | 108.5% | 120.4% | 0.0366 |
| TJR101392 | 31.1% | 67.1% | 81.3% | 0.0068 |
| TJR101393 | 34.1% | 73.3% | 80.3% | 0.0091 |

### Example 7. Inhibitory Activity of RNAi Agents Against Monkey APP in Primary Cynomolgus Monkey Hepatocytes (PCHs)

The RNAi agents in Table 10 were subjected to primary cynomolgus monkey hepatocyte (PCH) activity screening in primary cynomolgus monkey hepatocytes (PCHs) using 7 concentration gradients. The initial final concentration for transfection of each RNAi agent sample was 10 nM, and a 5-fold serial dilution was performed to obtain 7 concentration points.

PCHs were cryopreserved in liquid nitrogen. 24 h prior to transfection, the primary cynomolgus monkey hepatocytes (PCHs) were thawed and then seeded into a 96-well plate at a density of 3 × 10⁴ cells per well, with each well containing 80 µL of the culture medium.

The cells were transfected with the RNAi agents using Lipofectamine RNAi MAX (ThermoFisher, 13778150) according to the product instructions. The final gradient concentrations of the RNAi agents for transfection were 10 nM, 2 nM, 0.4 nM, 0.08 nM, 0.016 nM, 0.0032 nM, and 0.00064 nM. 24 h after treatment, total cell RNA extraction was performed using a high-throughput cell RNA extraction kit, and RNA reverse transcription and quantitative real-time PCR detection were performed to determine the mRNA level of monkey APP. The mRNA level of monkey APP was corrected based on the GAPDH internal reference gene level.

For the real-time quantitative PCR detection, a probe Q-PCR detection experiment was used. Information on the primers is shown in Table 11. The results of this example are shown in Table 12. The results show that the RNAi agents of the present disclosure exhibited high levels of on-target inhibitory activity against the APP gene in PCHs.

**Table 11. Taqman primers**

| Primer name | SEQ ID NO | Primer sequence (5'-3') |
|---|---|---|
| mkAPP-Forward | 245 | CCCACTTTGTGATTCCCTACC |
| mkAPP-Reverse | 246 | ATCCATCCTCTCCTGGTGTAA |
| mkAPP-Probe | 247 | 5`6-FAM- TAGTTGGTGAGTTTGTAAGCGATGCCC-3`BHQ1 |
| mkGAPDH-Forward | 248 | TCAAGATCGTCAGCAACGCC |
| mkGAPDH-Reverse | 249 | ACAGTCTTCTGGGTGGCAGT |
| mkGAPDH-Probe | 250 | 5`VIC-ACCAACTGCTTAGCACCCCTGGCCA-3`BHQ1 |

**Table 12. The activity of RNAi agents at 7 concentration points in PCHs (IC₅₀)**

| Remaining percentage of APP mRNA expression (mean) | | | | |
|---|---|---|---|---|
| Double strand code | 10.00000 nM | 2.00000 nM | 0.40000 nM | 0.08000 nM |
| TJR101344 | 10.3% | 8.7% | 10.6% | 13.4% |
| TJR101345 | 12.4% | 12.9% | 15.1% | 26.8% |
| TJR101383 | 26.9% | 22.9% | 31.8% | 54.3% |
| TJR101384 | 21.3% | 17.5% | 18.6% | 23.5% |
| TJR101391 | 31.9% | 28.3% | 30.3% | 45.1% |
| TJR101392 | 30.6% | 22.9% | 26.6% | 49.7% |
| TJR101393 | 36.9% | 25.3% | 32.6% | 54.2% |

| Double strand code | 0.01600 nM | 0.00320 nM | 0.00064 nM | IC50 (nM) |
|---|---|---|---|---|
| TJR101344 | 20.9% | 47.2% | 89.3% | 0.0028 |
| TJR101345 | 43.7% | 84.9% | 93.0% | 0.0143 |
| TJR101383 | 86.4% | 104.2% | 102.1% | 0.0966 |
| TJR101384 | 38.8% | 69.5% | 94.2% | 0.0082 |
| TJR101391 | 68.3% | 89.1% | 100.5% | 0.0492 |
| TJR101392 | 63.0% | 75.6% | 95.0% | 0.0444 |
| TJR101393 | 69.4% | 74.8% | 95.0% | 0.0778 |

### Example 8. Inhibitory Activity of RNAi Agents Against Human APP in A172 Cells

The RNAi agents of the present disclosure were subjected to an *in vitro* molecular-level activity assay in A172 cells using 7 concentration gradients. The initial final concentration for transfection of each RNAi agent sample was 10 nM, and a 7-fold serial dilution was performed to obtain 7 concentration points.

A172 cells were cultured at 37 °C with 5% CO₂ in a Dulbecco's modified eagle medium containing 10% fetal bovine serum. 24 h prior to transfection, the A172 cells were seeded into a 96-well plate at a density of 1 × 10⁴ cells per well, with each well containing 100 µL of the culture medium.

The cells were transfected with the RNAi agents using Lipofectamine RNAi MAX (ThermoFisher, 13778150) according to the product instructions. A total of 7 concentration points were set for the RNAi agents, the initial concentration was 10 nM, and two duplicate wells were set for each concentration. 48 h after treatment, total cell RNA extraction was performed using an MNTR/FX96 high-throughput cell RNA extraction kit (GeneOn BioTech, magnetic bead method), and RNA reverse transcription (Takara, RR037A) and quantitative real-time PCR (Thermo, 4444557) detection were performed to determine the mRNA level of human APP. The mRNA level of human APP was corrected based on the GAPDH internal reference gene level.

### Result analysis method

After the Q-PCR detection experiment was complete, corresponding Ct values were acquired according to a threshold value automatically set by the system, and the expression of a certain gene was relatively quantified by comparing the Ct values: comparing Ct refers to calculating differences in gene expression according to the differences from the Ct value of the internal reference gene and is also referred to as 2^{-△△Ct}, where △△Ct = [(target gene of Ct experimental group - internal reference of Ct experimental group) - (target gene of Ct control group - internal reference of Ct control group)]. Inhibition rate (%) = (1 - remaining level of target gene expression) × 100%.

The results are expressed relative to the remaining percentage of human APP mRNA expression in cells treated with the control RNAi agent. The inhibition rate IC₅₀ results are shown in Table 13. The results in Table 13 show that the RNAi agents of the present disclosure exhibited good inhibitory activity against APP mRNA.

**Table 13. The activity of RNAi agents at 7 concentration points in A172 cells (IC₅₀)**

| Double strand code | IC₅₀ (nM) |
|---|---|
| TJR103579 | 0.0087 |
| TJR103586 | 0.0064 |
| TJR103587 | 0.0265 |
| TJR103584 | 0.0019 |
| TJR103585 | 0.0018 |

### Example 9. psiCHECK On-Target Activity of RNAi Agents

The RNAi agents of the present disclosure were subjected to an *in vitro* molecular-level simulation of on-target activity screening in HEK293A cells using 9 concentration gradients.

On-target GSCM plasmids of the target sequence of the RNAi agents of the present disclosure were constructed and inserted into psiCHECK-2 plasmids. The plasmids contained the renilla luciferase gene and the firefly luciferase gene. In the dual reporter gene system, the target sequence of the RNAi agents was inserted into the 3' UTR region of the renilla luciferase gene. The activity of the RNAi agents for the target sequence can be reflected by measurement of renilla luciferase expression corrected with firefly luciferase.

HEK293A cells were cultured at 37 °C with 5% CO₂ in a DMEM high-glucose culture medium containing 10% fetal bovine serum. 24 h prior to transfection, the HEK293A cells were seeded into a 96-well plate at a density of 8 × 10³ cells per well, with each well containing 100 µL of the culture medium.

The cells were co-transfected with the RNAi agents and corresponding GSCM plasmids using Lipofectamine2000 (ThermoFisher, 11668019) according to the instructions, with 0.2 µL of Lipofectamine2000 used per well. The amount of plasmids for transfection was 20 ng per well. For the on-target sequence plasmids, a total of 9 concentration points were set. The initial concentration was 20 nM, and a 3-fold serial dilution was performed. Two duplicate wells were set for each concentration. 24 h after transfection, the on-target levels were determined using a Dual-Luciferase Reporter Assay System (Promega, E2940). The results are shown in Table 14. The results in Table 14 show that the RNAi agents of the present disclosure exhibited excellent inhibitory activity against APP mRNA.

**Table 14. The activity of RNAi agents at 9 concentration points in a psi-Check system (IC₅₀)**

| Double strand code | IC50 (nM) |
|---|---|
| TJR103580 | 0.0165 |
| TJR103589 | 0.1659 |
| TJR103581 | 0.0118 |
| TJR103592 | >20 |
| TJR103582 | 0.0206 |
| TJR103593 | >20 |
| TJR103583 | 0.005 |
| TJR103596 | 0.067 |
| TJR103778 | 0.030 |
| TJR103779 | 0.017 |
| TJR103780 | 0.007 |
| TJR103797 | 0.036 |
| TJR103798 | 0.072 |
| TJR103799 | 0.039 |

### Example 10. psiCHECK On-Target Activity of RNAi Agents

The RNAi agent sequences were subjected to an *in vitro* molecular-level simulation of on-target activity screening in HEK293A cells using 2 concentrations.

On-target GSCM plasmids of the target sequence of the RNAi agents were constructed and inserted into psiCHECK-2 plasmids. The plasmids contained the renilla luciferase gene and the firefly luciferase gene. In the dual reporter gene system, the target sequence of the RNAi agents was inserted into the 3' UTR region of the renilla luciferase gene. The activity of the RNAi agents for the target sequence can be reflected by measurement of renilla luciferase expression corrected with firefly luciferase.

HEK293A cells were cultured at 37 °C with 5% CO₂ in a DMEM high-glucose culture medium containing 10% fetal bovine serum. 24 h prior to transfection, the HEK293A cells were seeded into a 96-well plate at a density of 8 × 10³ cells per well, with each well containing 100 µL of the culture medium.

The cells were co-transfected with the RNAi agents and corresponding GSCM plasmids using Lipofectamine2000 (ThermoFisher, 11668019) according to the instructions, with 0.2 µL of Lipofectamine2000 used per well. The amount of plasmids for transfection was 20 ng per well. For the on-target sequence plasmids, a total of 2 concentration points were set: 0.03 nM and 0.01 nM, and two duplicate wells were set for each concentration. 24 h after transfection, the on-target levels were determined using a Dual-Luciferase Reporter Assay System (Promega, E2940).

The results are shown in Table 15. The results in Table 15 show that even at low concentrations, the RNAi agents of the present disclosure exhibited excellent inhibitory activity against APP mRNA in the psi-Check system.

**Table 15. The activity of RNAi agents at 2 concentration points in a psi-Check system**

| Double strand code | Remaining percentage of APP mRNA expression (mean) | |
|---|---|---|
| | 0.03 nM | 0.01 nM |
| TJR103596 | 48.84% | 57.67% |
| TJR103584 | 29.95% | 37.27% |
| TJR103585 | 26.95% | 32.29% |

### Example 11. Inhibitory Activity of RNAi Agents Against Human APP Gene Expression in A172 Cells

According to the method in Example 8, the compounds were subjected to an *in vitro* molecular-level activity assay in A172 cells using 8 concentration gradients. The initial final concentration for transfection of each RNAi agent sample was 10 nM, and a 7-fold serial dilution was performed to obtain 8 concentration points. The results are expressed relative to the remaining percentage of human APP mRNA expression in cells treated with the control RNAi agent. The inhibition rate IC₅₀ results are shown in Table 16. The results in Table 16 show that the RNAi agents of the present disclosure exhibited excellent inhibitory activity against APP mRNA.

**Table 16. The activity of RNAi agents at 8 concentration points in A172 cells (IC₅₀)**

| Double strand code | IC₅₀ (nM) |
|---|---|
| TJR103639 | 0.0206 |
| TJR103640 | 0.0624 |
| TJR103641 | 0.0560 |
| TJR103642 | 0.0282 |
| TJR103643 | 0.0643 |
| TJR103644 | 0.0435 |
| TJR103645 | 0.0166 |

## Claims

1. An RNAi agent, comprising a sense strand and an antisense strand that form a double-stranded region, wherein:
the sense strand comprises a sequence of at least 15 contiguous nucleotides differing from any one of the nucleotide sequences of SEQ ID NO: 1 to SEQ ID NO: 11 by no more than 3 nucleotides; and
the antisense strand comprises a sequence of at least 15 contiguous nucleotides differing from any one of the nucleotide sequences of SEQ ID NO: 12 to SEQ ID NO: 22 by no more than 3 nucleotides.

2. The RNAi agent according to claim 1, wherein the sense strand comprises a sequence of at least 17 contiguous nucleotides differing from any one of the nucleotide sequences of SEQ ID NO: 1 to SEQ ID NO: 11 by no more than 3 nucleotides, and the antisense strand comprises a sequence of at least 17 contiguous nucleotides differing from any one of the nucleotide sequences of SEQ ID NO: 12 to SEQ ID NO: 22 by no more than 3 nucleotides;
preferably, the sense strand comprises a sequence of at least 19 contiguous nucleotides differing from any one of the nucleotide sequences of SEQ ID NO: 1 to SEQ ID NO: 11 by no more than 3 nucleotides, preferably by no more than one nucleotide, and/or
the antisense strand comprises a sequence of at least 21 contiguous nucleotides differing from any one of the nucleotide sequences of SEQ ID NO: 12 to SEQ ID NO: 22 by no more than 3 nucleotides, preferably by no more than one nucleotide.

3. The RNAi agent according to claim 1 or 2, comprising or consisting of any one of the following combinations of sense and antisense strands: combination 1): a sense strand set forth in SEQ ID NO: 1 and an antisense strand set forth in SEQ ID NO: 12;
or a sense strand set forth in SEQ ID NO: 2 and an antisense strand set forth in SEQ ID NO: 13;
combination 2): a sense strand set forth in SEQ ID NO: 3 and an antisense strand set forth in SEQ ID NO: 14;
or a sense strand set forth in SEQ ID NO: 4 and an antisense strand set forth in SEQ ID NO: 15;
combination 3): a sense strand set forth in SEQ ID NO: 5 and an antisense strand set forth in SEQ ID NO: 16;
combination 4): a sense strand set forth in SEQ ID NO: 6 and an antisense strand set forth in SEQ ID NO: 17;
or a sense strand set forth in SEQ ID NO: 7 and an antisense strand set forth in SEQ ID NO: 18;
combination 6): a sense strand set forth in SEQ ID NO: 8 and an antisense strand set forth in SEQ ID NO: 19;
or a sense strand set forth in SEQ ID NO: 9 and an antisense strand set forth in SEQ ID NO: 20; and
combination 7): a sense strand set forth in SEQ ID NO: 10 and an antisense strand set forth in SEQ ID NO: 21;
or a sense strand set forth in SEQ ID NO: 11 and an antisense strand set forth in SEQ ID NO: 22.

4. The RNAi agent according to any one of claims 1-3, wherein at least one nucleotide in the sense strand and/or the antisense strand is a modified nucleotide;
preferably, all nucleotides in the sense strand and/or the antisense strand are modified nucleotides.

5. The RNAi agent according to claim 4, wherein three contiguous nucleotides in the sense strand are 2'-fluoro-modified nucleotides; and/or
in the direction from the 5' end to the 3' end, each of the nucleotides at positions 2, 6, 12, 14, and 16 of the antisense strand is independently a 2'-fluoro-modified nucleotide;
preferably,
in the direction from the 5' end to the 3' end, the nucleotides at positions 7, 8, and 9 of the sense strand are 2'-fluoro-modified nucleotides, and each of the nucleotides at positions 2, 6, 12, 14, and 16 of the antisense strand is independently a 2'-fluoro-modified nucleotide; or
in the direction from the 5' end to the 3' end, the nucleotides at positions 7, 8, and 9 of the sense strand are 2'-fluoro-modified nucleotides, and the nucleotide at position 2, 4, 6, 10, 12, 14, 16, or 18 of the antisense strand is independently a 2'-fluoro-modified nucleotide; and
the nucleotides at the remaining positions of the sense strand and the antisense strand are 2'-methoxy-modified nucleotides.

6. The RNAi agent according to any one of claims 1-5, wherein at least one phosphodiester group in the sense strand and/or the antisense strand is a phosphodiester group with a modification group;
preferably, the phosphodiester group with a modification group is a phosphorothioate diester group.

7. The RNAi agent according to claim 6, wherein the phosphodiester group with a modification group is present in at least one position selected from the group consisting of the following positions:
between the 1st and 2nd nucleotides of the 5' end of the sense strand;
between the 2nd and 3rd nucleotides of the 5' end of the sense strand;
between the 1st and 2nd nucleotides of the 3' end of the sense strand;
between the 2nd and 3rd nucleotides of the 3' end of the sense strand;
between the 1st and 2nd nucleotides of the 5' end of the antisense strand;
between the 2nd and 3rd nucleotides of the 5' end of the antisense strand;
between the 1st and 2nd nucleotides of the 3' end of the antisense strand; and
between the 2nd and 3rd nucleotides of the 3' end of the antisense strand.

8. The RNAi agent according to any one of claims 1-7, wherein the 5' end of the antisense strand comprises one phosphorus-containing group, and the phosphorus-containing group is a 5'-vinylphosphodiester group.

9. The RNAi agent according to any one of claims 1-8, wherein the RNAi agent is linked to one or more lipophilic groups;
preferably, the lipophilic group is linked to the 1st or 6th nucleotide of the 5' end of the sense strand.

10. The RNAi agent according to any one of claims 1-9, wherein the sense strand comprises the nucleotide sequence set forth in any one of SEQ ID NO: 109 to SEQ ID NO: 125;
and/or the antisense strand comprises the nucleotide sequence set forth in any one of SEQ ID NO: 126 to SEQ ID NO: 142;
preferably, the RNAi agent comprises or consists of any one of the following combinations of sense and antisense strands:
a sense strand set forth in SEQ ID NO: 109 and an antisense strand set forth in SEQ ID NO: 126;
a sense strand set forth in SEQ ID NO: 110 and an antisense strand set forth in SEQ ID NO: 127;
a sense strand set forth in SEQ ID NO: 111 and an antisense strand set forth in SEQ ID NO: 128;
a sense strand set forth in SEQ ID NO: 112 and an antisense strand set forth in SEQ ID NO: 129;
a sense strand set forth in SEQ ID NO: 113 and an antisense strand set forth in SEQ ID NO: 130;
a sense strand set forth in SEQ ID NO: 114 and an antisense strand set forth in SEQ ID NO: 131;
a sense strand set forth in SEQ ID NO: 115 and an antisense strand set forth in SEQ ID NO: 132;
a sense strand set forth in SEQ ID NO: 116 and an antisense strand set forth in SEQ ID NO: 133;
a sense strand set forth in SEQ ID NO: 117 and an antisense strand set forth in SEQ ID NO: 134;
a sense strand set forth in SEQ ID NO: 118 and an antisense strand set forth in SEQ ID NO: 135;
a sense strand set forth in SEQ ID NO: 119 and an antisense strand set forth in SEQ ID NO: 136;
a sense strand set forth in SEQ ID NO: 120 and an antisense strand set forth in SEQ ID NO: 137;
a sense strand set forth in SEQ ID NO: 121 and an antisense strand set forth in SEQ ID NO: 138;
a sense strand set forth in SEQ ID NO: 122 and an antisense strand set forth in SEQ ID NO: 139;
a sense strand set forth in SEQ ID NO: 123 and an antisense strand set forth in SEQ ID NO: 140;
a sense strand set forth in SEQ ID NO: 124 and an antisense strand set forth in SEQ ID NO: 141; and
a sense strand set forth in SEQ ID NO: 125 and an antisense strand set forth in SEQ ID NO: 142.

11. A pharmaceutical composition, comprising the RNAi agent according to any one of claims 1-10 and a pharmaceutically acceptable carrier.

12. A cell, comprising the RNAi agent according to any one of claims 1-10.

13. A kit, comprising the RNAi agent according to any one of claims 1-10 or the pharmaceutical composition according to claim 11.

14. A method for reducing amyloid precursor protein (APP) expression, comprising administering to a subject the RNAi agent according to any one of claims 1-10 or the pharmaceutical composition according to claim 11.

15. A method for treating and/or preventing a disease, comprising administering to a subject the RNAi agent according to any one of claims 1-10 or the pharmaceutical composition according to claim 11, wherein preferably, the disease is a neurodegenerative disease associated with the APP gene; more preferably, the disease is selected from the group consisting of Alzheimer's disease, frontotemporal dementia, and cerebral amyloid angiopathy.

16. A method for delivering an RNAi agent inhibiting APP expression and/or replication *in vivo*, comprising administering to a subject the RNAi agent according to any one of claims 1-10 or the pharmaceutical composition according to claim 11.

17. A method for preparing an RNAi agent or a pharmaceutical composition, comprising synthesizing the RNAi agent according to any one of claims 1-10 or the pharmaceutical composition according to claim 11.
